# EUROPEAN PATENT APPLICATION

(11) **EP 2 881 461 A1**
(43) Date of publication of application: **10.06.2015**
(21) Application number: 14192115.5
(22) Date of filing: 21.11.2008
(51) Int. Cl.: C12N 5/071

(54) **Reprogramming cells toward a pluripotent state**

(62) Divisional of application: 08400048.8
(71) Applicant: Fraunhofer-Gesellschaft zur Förderung der angewandten Forschung e.V., 80686 München (DE)
(72) Inventor: Stolzing, Alexandra, 04103 Leipzig (DE); Arnold, Antje, 04107 Leipzig (DE); Brown, Jeremy, Falmouth, Cornwall TR11 3EW (GB)
(74) Representative: Kilger, Ute

(57) **Abstract**

The present invention relates to a process for preparing reprogrammed cells, in particular for preparing pluripotent and multipotent stem cells and to the pluripotent and multipotent stem cells prepared by said processes.

SEQUENCE LISTING
<110> Fraunhofer-Gesellschaft zur Förderung der angewandten
Forschung e. V.
<120> Reprogramming cells toward a pluripotent state
<130> FBP25769-A
<160> 28
<170> PatentIn version 3.5
<210> 1
<211> 2098
<212> DNA
<213> Homo sapiens
<400> 1
attataaatc tagagactcc aggattttaa cgttctgctg gactgagctg gttgcctcat 60
gttattatgc aggcaactca ctttatccca atttcttgat acttttcctt ctggaggtcc 120
tatttctcta acatcttcca gaaaagtctt aaagctgcct taaccttttt tccagtccac 180
ctcttaaatt ttttcctcct cttcctctat actaacatga gtgtggatcc agcttgtccc 240
caaagcttgc cttgctttga agcatccgac tgtaaagaat cttcacctat gcctgtgatt 300
tgtgggcctg aagaaaacta tccatccttg caaatgtctt ctgctgagat gcctcacacg 360
gagactgtct ctcctcttcc ttcctccatg gatctgctta ttcaggacag ccctgattct 420
tccaccagtc ccaaaggcaa acaacccact tctgcagaga agagtgtcgc aaaaaaggaa 480
gacaaggtcc cggtcaagaa acagaagacc agaactgtgt tctcttccac ccagctgtgt 540
gtactcaatg atagatttca gagacagaaa tacctcagcc tccagcagat gcaagaactc 600
tccaacatcc tgaacctcag ctacaaacag gtgaagacct ggttccagaa ccagagaatg 660
aaatctaaga ggtggcagaa aaacaactgg ccgaagaata gcaatggtgt gacgcagaag 720
gcctcagcac ctacctaccc cagcctttac tcttcctacc accagggatg cctggtgaac 780
ccgactggga accttccaat gtggagcaac cagacctgga acaattcaac ctggagcaac 840
cagacccaga acatccagtc ctggagcaac cactcctgga acactcagac ctggtgcacc 900
caatcctgga acaatcaggc ctggaacagt cccttctata actgtggaga ggaatctctg 960
cagtcctgca tgcagttcca gccaaattct cctgccagtg acttggaggc tgccttggaa 1020
gctgctgggg aaggccttaa tgtaatacag cagaccacta ggtattttag tactccacaa 1080
accatggatt tattcctaaa ctactccatg aacatgcaac ctgaagacgt gtgaagatga 1140
gtgaaactga tattactcaa tttcagtctg gacactggct gaatccttcc tctcccctcc 1200
tcccatccct cataggattt ttcttgtttg gaaaccacgt gttctggttt ccatgatgcc 1260
catccagtca atctcatgga gggtggagta tggttggagc ctaatcagcg aggtttcttt 1320
tttttttttt ttcctattgg atcttcctgg agaaaatact tttttttttt ttttttttga 1380
aacggagtct tgctctgtcg cccaggctgg agtgcagtgg cgcggtcttg gctcactgca 1440
agctccgtct cccgggttca cgccattctc ctgcctcagc ctcccgagca gctgggacta 1500
caggcgcccg ccacctcgcc cggctaatat tttgtatttt tagtagagac ggggtttcac 1560
tgtgttagcc aggatggtct cgatctcctg accttgtgat ccacccgcct cggcctccct 1620
aacagctggg atttacaggc gtgagccacc gcgccctgcc tagaaaagac attttaataa 1680
ccttggctgc cgtctctggc tatagataag tagatctaat actagtttgg atatctttag 1740
ggtttagaat ctaacctcaa gaataagaaa tacaagtaca aattggtgat gaagatgtat 1800
tcgtattgtt tgggattggg aggctttgct tattttttaa aaactattga ggtaaagggt 1860
taagctgtaa catacttaat tgatttctta ccgtttttgg ctctgttttg ctatatcccc 1920
taatttgttg gttgtgctaa tctttgtaga aagaggtctc gtatttgctg catcgtaatg 1980
acatgagtac tgctttagtt ggtttaagtt caaatgaatg aaacaactat tttcctttta 2040
gttgatttta ccctgatttc accgagtgtt tcaatgagta aatatacagc ttaaacat 2098

<210> 2
<211> 1356
<212> DNA
<213> Mus musculus
<400> 2
tctatcgcct tgagccgttg gccttcagat aggctgattt ggttggtgtc ttgctctttc 60
tgtgggaagg ctgcggctca cttccttctg acttcttgat aattttgcat tagacattta 120
actcttcttt ctatgatctt tccttctaga cactgagttt tttggttgtt gcctaaaacc 180
ttttcagaaa tcccttccct cgccatcaca ctgacatgag tgtgggtctt cctggtcccc 240
acagtttgcc tagttctgag gaagcatcga attctgggaa cgcctcatca atgcctgcag 300
tttttcatcc cgagaactat tcttgcttac aagggtctgc tactgagatg ctctgcacag 360
aggctgcctc tcctcgccct tcctctgaag acctgcctct tcaaggcagc cctgattctt 420
ctaccagtcc caaacaaaag ctctcaagtc ctgaggctga caagggccct gaggaggagg 480
agaacaaggt ccttgccagg aagcagaaga tgcggactgt gttctctcag gcccagctgt 540
gtgcactcaa ggacaggttt cagaagcaga agtacctcag cctccagcag atgcaagaac 600

tctcctccat tctgaacctg agctataagc aggttaagac ctggtttcaa aaccaaagga 660
tgaagtgcaa gcggtggcag aaaaaccagt ggttgaagac tagcaatggt ctgattcaga 720
agggctcagc accagtggag tatcccagca tccattgcag ctatccccag ggctatctgg 780
tgaacgcatc tggaagcctt tccatgtggg gcagccagac ttggaccaac ccaacttgga 840
gcagccagac ctggaccaac ccaacttgga acaaccagac ctggaccaac ccaacttgga 900
gcagccaggc ctggaccgct cagtcctgga acggccagcc ttggaatgct gctccgctcc 960
ataacttcgg ggaggacttt ctgcagcctt acgtacagtt gcagcaaaac ttctctgcca 1020
gtgatttgga ggtgaatttg gaagccacta gggaaagcca tgcgcatttt agcaccccac 1080
aagccttgga attattcctg aactactctg tgactccacc aggtgaaata tgagacttac 1140
gcaacatctg ggcttaaagt cagggcaaag ccaggttcct tccttcttcc aaatattttc 1200
atattttttt taaagattta tttattcatt atatgtaagt acactgtagc tgtcttcaga 1260
cactccagaa gagggcgtca gatcttgtta cgtatggttg tgagccacca tgtggttgct 1320
gggatttgaa ctcctgacct tcggaagagc agtcgg 1356

<210> 3
<211> 2358
<212> DNA
<213> Rattus norvegicus
<400> 3
   tcagataggc tgatttcgag tctttctctt ttgtgggaag accgaggctc gcttcttttt 60
   ggcttgttga ctcttttaca tctggacatt taactcttac ttttaagatc tttccctcta 120
   gacactgagt tttaaagtct taactttttg gttgttaaaa actttttttt ttttaaagtc 180
   ccttcccttg ccgttgggct gacatgagcg tggatctttc tggtccccac agtctgccta 240
   gttgtgagga agcatcgaac tctggggatt cctcgccgat gcctgccgtt catcttcctg 300
   aggaaaatta ttcttgctta caagtgtctg ctactgagat gctctgcaca gagactgcct 360
   ctcctccgcc ttcctctggg gacctacctc ttcaagatag ccctgattct tctagcaatc 420
   ccaagctaaa gctgtctggt cccgaggctg acgagggccc tgagaagaaa gaagagaaca 480
   aggtcctcac caagaagcag aagatgcgga ctgtgttctc tcaggcccag ttgtgtgcac 540
   tcaaggatag gtttcagagg caaaggtacc tcagcctcca gcagatgcaa gatctctcta 600
   ccattctgaa cctgagctat aagcaggtga agacctggtt ccaaaaccaa agaatgaagt 660
   gcaagaggtg gcagaaaaac caatggttga agactagcaa cggcctgact cagaagggct 720
   cagcgccggt ggagtatccc agcatccatt gcagctattc tcagggctat ctgatgaacg 780
   cgtctggaaa ccttccagta tggggcagtc agacctggac caacccaact tggaacaacc 840
   agacctggac caacccaacc tggagcaacc agacctggac caacccaact tggagcaacc 900
   aggcctggag cactcagtcc tggtgtactc aggcctggaa cagccagact tggaacgctg 960
   ctccgctcca taacttcggg gaggactccc tgcagcctta tgtgccgttg cagcaaaact 1020
   tctccgccag tgatttggag gcgaatttgg aagccactag ggaaagccag gcgcatttta 1080
   gtaccccgca agccttggaa ttgttcctga actactccgt gaattctcca ggcgaaatat 1140
   gaggtttaca caacaactgg gcttaaagtc agggcagggc cagggtcagc tttcttcctt 1200
   cttccaaaga gttttatatt gttcttattt ttttttaaat tattattttg tttttgtttt 1260
   ttgtttatca aggtagggtt tctctgtgtg gttctggctg tcctggaatt cactctgtag 1320
   accaggctgg cctcgtactc agagatctgc ctacttttgc ctcctgaagg ctagggctaa 1380
   agattttcta aagattttca tagtttttat ttttttaatt attatctgtt ttcatgtttg 1440
   tgtttttttg ttttgttttt gtttttgttt ttgtttatca agatagggtt tctctgtgtg 1500
   gctctagtag tcccggaaac tggctctgta gaccaggctg tccttgaact cagaaatctg 1560
   cctttgcctc cggactgcgg ggactaaagg cagtatataa ccacctggca cattgttttt 1620
   atttttattc ttttggtgcc agaaagcaaa cctaggactt tgagctgggc acccactcaa 1680
   ccactgagct ctgtttgcga cccccgtgtt ggctgcattt gtctgagctg ggtaacttgt 1740
   ctttttttcc gtgttaacga tgggcttcgg agacagtgca ctatacactc tatcctcccc 1800
   caggtctcac acacccaccc tactccatac caacccaggc ttgtctgtct tttttttttt 1860
   tggagctgag gactgaaccc agggccttgc gctttctagg caagcgctct acctctgagc 1920
   taaatcccca acccttgtct gtctttttag aagcttgggt cttggtgtgc actgtgtatc 1980
   gttttgaggg gtgaggttta aaagtataca aattataaag attcatgcag atatggtggc 2040
   tcctctcaag gacgagacag aaggatcacc agtttgaggc tatctcagat ataaaataag 2100
   ttcaagacca gcctgtacta tgtctaaata gtaagacagc atctcaacaa aataataaaa 2160
   ctaaggtaag gagataaaag taaagtctca acaaaataca agatctcgcc tgttacagtt 2220
   ctttgatttc ctccgtgtct ttgcagttcc gccaagaggc ttctatgtta atatctgtag 2280
   aaagatgttt atatttgact gtaccatgat aaaccagtgc cagctggact agtttaaata 2340
   aaacactaat tttaccca 2358

<210> 4
<211> 2518
<212> DNA

<213> Homo sapiens
<400> 4
   ctattaactt gttcaaaaaa gtatcaggag ttgtcaaggc agagaagaga gtgtttgcaa 60
   aagggggaaa gtagtttgct gcctctttaa gactaggact gagagaaaga agaggagaga 120
   gaaagaaagg gagagaagtt tgagccccag gcttaagcct ttccaaaaaa taataataac 180
   aatcatcggc ggcggcagga tcggccagag gaggagggaa gcgctttttt tgatcctgat 240
   tccagtttgc ctctctcttt ttttccccca aattattctt cgcctgattt tcctcgcgga 300
   gccctgcgct cccgacaccc ccgcccgcct cccctcctcc tctccccccg cccgcgggcc 360
   ccccaaagtc ccggccgggc cgagggtcgg cggccgccgg cgggccgggc ccgcgcacag 420
   cgcccgcatg tacaacatga tggagacgga gctgaagccg ccgggcccgc agcaaacttc 480
   ggggggcggc ggcggcaact ccaccgcggc ggcggccggc ggcaaccaga aaaacagccc 540
   ggaccgcgtc aagcggccca tgaatgcctt catggtgtgg tcccgcgggc agcggcgcaa 600
   gatggcccag gagaacccca agatgcacaa ctcggagatc agcaagcgcc tgggcgccga 660
   gtggaaactt ttgtcggaga cggagaagcg gccgttcatc gacgaggcta agcggctgcg 720
   agcgctgcac atgaaggagc acccggatta taaataccgg ccccggcgga aaaccaagac 780
   gctcatgaag aaggataagt acacgctgcc cggcgggctg ctggcccccg gcggcaatag 840
   catggcgagc ggggtcgggg tgggcgccgg cctgggcgcg ggcgtgaacc agcgcatgga 900
   cagttacgcg cacatgaacg gctggagcaa cggcagctac agcatgatgc aggaccagct 960
   gggctacccg cagcacccgg gcctcaatgc gcacggcgca gcgcagatgc agcccatgca 1020
   ccgctacgac gtgagcgccc tgcagtacaa ctccatgacc agctcgcaga cctacatgaa 1080
   cggctcgccc acctacagca tgtcctactc gcagcagggc acccctggca tggctcttgg 1140
   ctccatgggt tcggtggtca agtccgaggc cagctccagc ccccctgtgg ttacctcttc 1200
   ctcccactcc agggcgccct gccaggccgg ggacctccgg gacatgatca gcatgtatct 1260
   ccccggcgcc gaggtgccgg aacccgccgc ccccagcaga cttcacatgt cccagcacta 1320
   ccagagcggc ccggtgcccg gcacggccat taacggcaca ctgcccctct cacacatgtg 1380
   agggccggac agcgaactgg aggggggaga aattttcaaa gaaaaacgag ggaaatggga 1440
   ggggtgcaaa agaggagagt aagaaacagc atggagaaaa cccggtacgc tcaaaaagaa 1500
   aaaggaaaaa aaaaaatccc atcacccaca gcaaatgaca gctgcaaaag agaacaccaa 1560
   tcccatccac actcacgcaa aaaccgcgat gccgacaaga aaacttttat gagagagatc 1620
   ctggacttct tttgggggga ctatttttgt acagagaaaa cctggggagg gtggggaggg 1680
   cgggggaatg gaccttgtat agatctggag gaaagaaagc tacgaaaaac tttttaaaag 1740
   ttctagtggt acggtaggag ctttgcagga agtttgcaaa agtctttacc aataatattt 1800
   agagctagtc tccaagcgac gaaaaaaatg ttttaatatt tgcaagcaac ttttgtacag 1860
   tatttatcga gataaacatg gcaatcaaaa tgtccattgt ttataagctg agaatttgcc 1920
   aatatttttc aaggagaggc ttcttgctga attttgattc tgcagctgaa atttaggaca 1980
   gttgcaaacg tgaaaagaag aaaattattc aaatttggac attttaattg tttaaaaatt 2040
   gtacaaaagg aaaaaattag aataagtact ggcgaaccat ctctgtggtc ttgtttaaaa 2100
   agggcaaaag ttttagactg tactaaattt tataacttac tgttaaaagc aaaaatggcc 2160
   atgcaggttg acaccgttgg taatttataa tagcttttgt tcgatcccaa ctttccattt 2220
   tgttcagata aaaaaaacca tgaaattact gtgtttgaaa tattttctta tggtttgtaa 2280
   tatttctgta aatttattgt gatattttaa ggttttcccc cctttatttt ccgtagttgt 2340
   attttaaaag attcggctct gtattatttg aatcagtctg ccgagaatcc atgtatatat 2400
   ttgaactaat atcatcctta taacaggtac attttcaact taagttttta ctccattatg 2460
   cacagtttga gataaataaa tttttgaaat atggacactg aaaaaaaaaa aaaaaaaa 2518

<210> 5
<211> 2457
<212> DNA
<213> Mus musculus
<400> 5
ctattaactt gttcaaaaaa gtatcaggag ttgtcaaggc agagaagaga gtgtttgcaa 60
aaagggaaaa gtactttgct gcctctttaa gactagggct gggagaaaga agaggagaga 120
gaaagaaagg agagaagttt ggagcccgag gcttaagcct ttccaaaaac taatcacaac 180
aatcgcggcg gcccgaggag gagagcgcct gttttttcat cccaattgca cttcgcccgt 240
ctcgagctcc gcttcccccc aactattctc cgccagatct ccgcgcaggg ccgtgcacgc 300
cgaggccccc gcccgcggcc cctgcatccc ggcccccgag cgcggccccc acagtcccgg 360
ccgggccgag ggttggcggc cgccggcggg ccgcgcccgc ccagcgcccg catgtataac 420
atgatggaga cggagctgaa gccgccgggc ccgcagcaag cttcgggggg cggcggcgga 480
ggaggcaacg ccacggcggc ggcgaccggc ggcaaccaga agaacagccc ggaccgcgtc 540
aagaggccca tgaacgcctt catggtatgg tcccgggggc agcggcgtaa gatggcccag 600
gagaacccca agatgcacaa ctcggagatc agcaagcgcc tgggcgcgga gtggaaactt 660
ttgtccgaga ccgagaagcg gccgttcatc gacgaggcca agcggctgcg cgctctgcac 720
atgaaggagc acccggatta taaataccgg ccgcggcgga aaaccaagac gctcatgaag 780

aaggataagt acacgcttcc cggaggcttg ctggcccccg gcgggaacag catggcgagc 840
ggggttgggg tgggcgccgg cctgggtgcg ggcgtgaacc agcgcatgga cagctacgcg 900
cacatgaacg gctggagcaa cggcagctac agcatgatgc aggagcagct gggctacccg 960
cagcacccgg gcctcaacgc tcacggcgcg gcacagatgc aaccgatgca ccgctacgac 1020
gtcagcgccc tgcagtacaa ctccatgacc agctcgcaga cctacatgaa cggctcgccc 1080
acctacagca tgtcctactc gcagcagggc acccccggta tggcgctggg ctccatgggc 1140
tctgtggtca agtccgaggc cagctccagc ccccccgtgg ttacctcttc ctcccactcc 1200
agggcgccct gccaggccgg ggacctccgg gacatgatca gcatgtacct ccccggcgcc 1260
gaggtgccgg agcccgctgc gcccagtaga ctgcacatgg cccagcacta ccagagcggc 1320
ccggtgcccg gcacggccat taacggcaca ctgcccctgt cgcacatgtg agggctggac 1380
tgcgaactgg agaaggggag agattttcaa agagatacaa gggaattggg aggggtgcaa 1440
aaagaggaga gtaggaaaaa tctgataatg ctcaaaagga aaaaaaatct ccgcagcgaa 1500
acgacagctg cggaaaaaaa ccaccaatcc catccaaatt aacgcaaaaa ccgtgatgcc 1560
gactagaaaa cttttatgag agatcttggg acttcttttt gggggactat ttttgtacag 1620
agaaaacctg agggcggcgg ggagggcggg ggaatcggac catgtataga tctggaggaa 1680
aaaaactacg caaaactttt ttttaaagtt ctagtggtac gttaggcgct tcgcagggag 1740
ttcgcaaaag tctttaccag taatatttag agctagactc cgggcgatga aaaaaaagtt 1800
ttaatatttg caagcaactt ttgtacagta tttatcgaga taaacatggc aatcaaatgt 1860
ccattgttta taagctgaga atttgccaat atttttcgag gaaagggttc ttgctgggtt 1920
ttgattctgc agcttaaatt taggaccgtt acaaacaagg aaggagttta ttcggatttg 1980
aacattttag ttttaaaatt gtacaaaagg aaaacatgag agcaagtact ggcaagaccg 2040
ttttcgtggt cttgtttaag gcaaacgttc tagattgtac taaattttta acttactgtt 2100
aaaggcaaaa aaaaaatgtc catgcaggtt gatatcgttg gtaatttata atagcttttg 2160
ttcaatccta ccctttcatt ttgttcacat aaaaaatatg gaattactgt gtttgaaata 2220
ttttcttatg gtttgtaata tttctgtaaa ttgtgatatt ttaaggtttt tccccccttt 2280
tattttccgt agttgtattt taaaagattc ggctctgtta ttggaatcag gctgccgaga 2340
atccatgtat atatttgaac taataccatc cttataacag ctacattttc aacttaagtt 2400
tttactccat tatgcacagt ttgagataaa taaatttttg aaatatggac actgaaa 2457

<210> 6
<211> 2323
<212> DNA
<213> Rattus norvegicus
<400> 6
gtgtttgcaa aaagggaaaa gtactttgct gcctctttaa gactagggct gggagaaaga 60
agaggagaga aaaagaaagg agagaagttt ggagcccgag gcttaagcct ttccaaaaac 120
taatcacaac aatcgcggcg gcccgaggag gagagcgact gttttttcat cccaattgca 180
cttcgcccgt ctcgagctcc gcttcccccc aactattctc cgccagatct ccgcgcaagg 240
ccgtgcacgc cgacgacccc gcccgcggcc cctgcatccc ggcccccgcg cgcggccccc 300
gcagtcccgg ccgggccgag ggtcggcggc cgccggcggg ccgcgcccgc gcccagcgcc 360
cgcatgtata acatgatgga gacggagctg aagccgccgg gccctcagca agcttcgggg 420
ggcggcggcg gaggaggcaa cgccacggcg gcggcgaccg gcggcaacca gaagaacagc 480
ccggaccgcg tcaagaggcc catgaatgcc ttcatggtgt ggtcccgggg gcagcggcgt 540
aagatggccc aggagaaccc caagatgcac aactcggaga tcagcaagcg cctgggcgcc 600
gagtggaaac ttttgtcgga gaccgagaag cggccgttca tcgacgaggc caagcggctg 660
cgcgctctgc acatgaagga gcacccggat tataaatacc ggccgcggcg gaaaaccaag 720
acgctcatga agaaggataa gtacacgctt cccggaggct tgctggcccc cggcgggaac 780
agcatggcga gcggggttgg ggtgggcgcc ggcctgggtg cgggcgtgaa ccagcgcatg 840
gacagctacg cgcacatgaa cggctggagc aacggcagct acagcatgat gcaggagcag 900
ctgggctacc cgcagcaccc gggcctcaac gctcacggcg cggcacagat gcagccgatg 960
caccgctacg acgtcagcgc cctgcagtac aactccatga ccagctcgca gacctacatg 1020
aacggctcgc ccacctacag catgtcctac tcgcagcagg gcacccccgg tatggcgctg 1080
ggctccatgg gctctgtggt caagtccgag gccagttcca gcccccccgt ggttacctct 1140
tcctcccact ccagggcgcc ctgccaggcc ggggacctcc gggacatgat cagcatgtac 1200
ctccccggcg ccgaggtgcc ggagcccgct gcgcccagta gactgcacat ggcccagcac 1260
taccagagcg gcccggtgcc cggcacggcc attaacggca cactgcccct gtcgcacatg 1320
tgagggccgg accgcgaact ggagaagggg agagattttt caaaaagata caagggaatt 1380
gggaggggtg caaaagagga gagtaagaaa aatctgaatg ctcaaaagga aaaaaaaaat 1440
ctcattaccc gcagcaaaat gacagctgcg gaaaaaaacc accaatccca tccaaattaa 1500
cgcaaaaacc gtgatgccga ctagaaaact tttatgagag atctggagga aaaaaactac 1560
gcaaaacttt tttttaaagt tctagtggta cgttaggcgc ttcgcaggga gttctcaaaa 1620
gtctttacca gtaatattta gaactagact ccgggcgatg aaaaaagttt taatatttgc 1680
aagcaacttt tgtacagtat ttatcgagat aaacatggca atcaaatgtc cattgtttat 1740

aagctgagaa tttgccaata tttttcgagg aaagggttct tgctgggttt tgattctgca 1800
gcttaaatta aggaccgtta cagacaagga aggaatttat tcggatttga acgttttagt 1860
tttaaaattg tacaaaagga aaacatgaga gcaagtactg gcaagaccat tttcgtggtc 1920
ttgtttaggg caaacgttct agattgtact aaatttttaa cttactgtta aaggcaaaaa 1980
aaaaatgtcc atgcaggttg atatcgttgg taatttataa tagcttttgt tcaatcccac 2040
ccttttcatt ttgttcacat aaaaatatgg aaattactgt gtttgaaata ttttcttatg 2100
gtttgtaata tttctgtaaa ttgtgatatt ttaaggtttt ttcccccttt tattttccgt 2160
agttgtattt taaaagattc ggctgttatt ggaaccaggc tgccgagaat ccatgtatat 2220
atttgaacta ataccatcct tataacagtt acgtttccaa cttaagtttt tactccatta 2280
tgcacagttt gagataaata aatttttgaa atatggacac tga 2323

<210> 7
<211> 1411
<212> DNA
<213> Homo sapiens
<400> 7
ccttcgcaag ccctcatttc accaggcccc cggcttgggg cgccttcctt ccccatggcg 60
ggacacctgg cttcggattt cgccttctcg ccccctccag gtggtggagg tgatgggcca 120
ggggggccgg agccgggctg ggttgatcct cggacctggc taagcttcca aggccctcct 180
ggagggccag gaatcgggcc gggggttggg ccaggctctg aggtgtgggg gattccccca 240
tgccccccgc cgtatgagtt ctgtgggggg atggcgtact gtgggcccca ggttggagtg 300
gggctagtgc cccaaggcgg cttggagacc tctcagcctg agggcgaagc aggagtcggg 360
gtggagagca actccgatgg ggcctccccg gagccctgca ccgtcacccc tggtgccgtg 420
aagctggaga aggagaagct ggagcaaaac ccggaggagt cccaggacat caaagctctg 480
cagaaagaac tcgagcaatt tgccaagctc ctgaagcaga agaggatcac cctgggatat 540
acacaggccg atgtggggct caccctgggg gttctatttg ggaaggtatt cagccaaacg 600
accatctgcc gctttgaggc tctgcagctt agcttcaaga acatgtgtaa gctgcggccc 660
ttgctgcaga agtgggtgga ggaagctgac aacaatgaaa atcttcagga gatatgcaaa 720
gcagaaaccc tcgtgcaggc ccgaaagaga aagcgaacca gtatcgagaa ccgagtgaga 780
ggcaacctgg agaatttgtt cctgcagtgc ccgaaaccca cactgcagca gatcagccac 840
atcgcccagc agcttgggct cgagaaggat gtggtccgag tgtggttctg taaccggcgc 900
cagaaggcca agcgatcaag cagcgactat gcacaacgag aggattttga ggctgctggg 960
tctcctttct cagggggacc agtgtccttt cctctggccc cagggcccca ttttggtacc 1020
ccaggctatg ggagccctca cttcactgca ctgtactcct cggtcccttt ccctgagggg 1080
gaagcctttc cccctgtctc cgtcaccact ctgggctctc ccatgcattc aaactgaggt 1140
gcctgccctt ctaggaatgg gggacagggg gaggggagga gctagggaaa gaaaacctgg 1200
agtttgtgcc agggtttttg ggattaagtt cttcattcac taaggaagga attgggaaca 1260
caaagggtgg gggcagggga gtttggggca actggttgga gggaaggtga agttcaatga 1320
tgctcttgat tttaatccca catcatgtat cacttttttc ttaaataaag aagcctggga 1380
cacagtagat agacacactt aaaaaaaaaa a 1411

<210> 8
<211> 1346
<212> DNA
<213> Mus musculus
<400> 8
aaccgtccct aggtgagccg tctttccacc aggcccccgg ctcggggtgc ccaccttccc 60
catggctgga cacctggctt cagacttcgc cttctcaccc ccaccaggtg ggggtgatgg 120
gtcagcaggg ctggagccgg gctgggtgga tcctcgaacc tggctaagct tccaaggccc 180
tccaggtggg cctggaatcg gaccaggctc agaggtattg gggatctccc catgtccgcc 240
cgcatacgag ttctgcggag ggatggcata ctgtggacct caggttggac tgggcctagt 300
cccccaagtt ggcgtggaga ctttgcagcc tgagggccag gcaggagcac gagtggaaag 360
caactcagag ggaacctcct ctgagccctg tgccgaccgc cccaatgccg tgaagttgga 420
gaaggtggaa ccaactcccg aggagtccca ggacatgaaa gccctgcaga aggagctaga 480
acagtttgcc aagctgctga agcagaagag gatcaccttg gggtacaccc aggccgacgt 540
ggggctcacc ctgggcgttc tctttggaaa ggtgttcagc cagaccacca tctgtcgctt 600
cgaggccttg cagctcagcc ttaagaacat gtgtaagctg cggcccctgc tggagaagtg 660
ggtggaggaa gccgacaaca atgagaacct tcaggagata tgcaaatcgg agaccctggt 720
gcaggcccgg aagagaaagc gaactagcat tgagaaccgt gtgaggtgga gtctggagac 780
catgtttctg aagtgcccga agccctccct acagcagatc actcacatcg ccaatcagct 840
tgggctagag aaggatgtgg ttcgagtatg gttctgtaac cggcgccaga agggcaaaag 900
atcaagtatt gagtattccc aacgagaaga gtatgaggct acagggacac ctttcccagg 960
gggggctgta tcctttcctc tgcccccagg tccccacttt ggcaccccag gctatggaag 1020

cccccacttc accacactct actcagtccc ttttcctgag ggcgaggcct ttccctctgt 1080
tcccgtcact gctctgggct ctcccatgca ttcaaactga ggcaccagcc ctccctgggg 1140
atgctgtgag ccaaggcaag ggaggtagac aagagaacct ggagctttgg ggttaaattc 1200
ttttactgag gagggattaa aagcacaaca ggggtggggg gtgggatggg gaaagaagct 1260
cagtgatgct gttgatcagg agcctggcct gtctgtcact catcattttg ttcttaaata 1320
aagactggga cacacagtag atagct 1346

<210> 9
<211> 2949
<212> DNA
<213> Homo sapiens
<400> 9
agtttcccga ccagagagaa cgaacgtgtc tgcgggcgcg cggggagcag aggcggtggc 60
gggcggcggc ggcaccggga gccgccgagt gaccctcccc cgcccctctg gccccccacc 120
ctcccacccg cccgtggccc gcgcccatgg ccgcgcgcgc tccacacaac tcaccggagt 180
ccgcgccttg cgccgccgac cagttcgcag ctccgcgcca cggcagccag tctcacctgg 240
cggcaccgcc cgcccaccgc cccggccaca gcccctgcgc ccacggcagc actcgaggcg 300
accgcgacag tggtggggga cgctgctgag tggaagagag cgcagcccgg ccaccggacc 360
tacttactcg ccttgctgat tgtctatttt tgcgtttaca acttttctaa gaacttttgt 420
atacaaagga actttttaaa aaagacgctt ccaagttata tttaatccaa agaagaagga 480
tctcggccaa tttggggttt tgggttttgg cttcgtttct tctcttcgtt gactttgggg 540
ttcaggtgcc ccagctgctt cgggctgccg aggaccttct gggcccccac attaatgagg 600
cagccacctg gcgagtctga catggctgtc agcgacgcgc tgctcccatc tttctccacg 660
ttcgcgtctg gcccggcggg aagggagaag acactgcgtc aagcaggtgc cccgaataac 720
cgctggcggg aggagctctc ccacatgaag cgacttcccc cagtgcttcc cggccgcccc 780
tatgacctgg cggcggcgac cgtggccaca gacctggaga gcggcggagc cggtgcggct 840
tgcggcggta gcaacctggc gcccctacct cggagagaga ccgaggagtt caacgatctc 900
ctggacctgg actttattct ctccaattcg ctgacccatc ctccggagtc agtggccgcc 960
accgtgtcct cgtcagcgtc agcctcctct tcgtcgtcgc cgtcgagcag cggccctgcc 1020
agcgcgccct ccacctgcag cttcacctat ccgatccggg ccgggaacga cccgggcgtg 1080
gcgccgggcg gcacgggcgg aggcctcctc tatggcaggg agtccgctcc ccctccgacg 1140
gctcccttca acctggcgga catcaacgac gtgagcccct cgggcggctt cgtggccgag 1200
ctcctgcggc cagaattgga cccggtgtac attccgccgc agcagccgca gccgccaggt 1260
ggcgggctga tgggcaagtt cgtgctgaag gcgtcgctga gcgcccctgg cagcgagtac 1320
ggcagcccgt cggtcatcag cgtcagcaaa ggcagccctg acggcagcca cccggtggtg 1380
gtggcgccct acaacggcgg gccgccgcgc acgtgcccca agatcaagca ggaggcggtc 1440
tcttcgtgca cccacttggg cgctggaccc cctctcagca atggccaccg gccggctgca 1500
cacgacttcc ccctggggcg gcagctcccc agcaggacta ccccgaccct gggtcttgag 1560
gaagtgctga gcagcaggga ctgtcaccct gccctgccgc ttcctcccgg cttccatccc 1620
cacccggggc ccaattaccc atccttcctg cccgatcaga tgcagccgca agtcccgccg 1680
ctccattacc aagagctcat gccacccggt tcctgcatgc cagaggagcc caagccaaag 1740
aggggaagac gatcgtggcc ccggaaaagg accgccaccc acacttgtga ttacgcgggc 1800
tgcggcaaaa cctacacaaa gagttcccat ctcaaggcac acctgcgaac ccacacaggt 1860
gagaaacctt accactgtga ctgggacggc tgtggatgga aattcgcccg ctcagatgaa 1920
ctgaccaggc actaccgtaa acacacgggg caccgcccgt tccagtgcca aaaatgcgac 1980
cgagcatttt ccaggtcgga ccacctcgcc ttacacatga agaggcattt ttaaatccca 2040
gacagtggat atgacccaca ctgccagaag agaattcagt attttttact tttcacactg 2100
tcttcccgat gagggaagga gcccagccag aaagcactac aatcatggtc aagttcccaa 2160
ctgagtcatc ttgtgagtgg ataatcagga aaaatgagga atccaaaaga caaaaatcaa 2220
agaacagatg gggtctgtga ctggatcttc tatcattcca attctaaatc cgacttgaat 2280
attcctggac ttacaaaatg ccaagggggt gactggaagt tgtggatatc agggtataaa 2340
ttatatccgt gagttggggg agggaagacc agaattccct tgaattgtgt attgatgcaa 2400
tataagcata aaagatcacc ttgtattctc tttaccttct aaaagccatt attatgatgt 2460
tagaagaaga ggaagaaatt caggtacaga aaacatgttt aaatagccta aatgatggtg 2520
cttggtgagt cttggttcta aaggtaccaa acaaggaagc caaagttttc aaactgctgc 2580
atactttgac aaggaaaatc tatatttgtc ttccgatcaa catttatgac ctaagtcagg 2640
taatatacct ggtttacttc tttagcattt ttatgcagac agtctgttat gcactgtggt 2700
ttcagatgtg caataatttg tacaatggtt tattcccaag tatgccttaa gcagaacaaa 2760
tgtgtttttc tatatagttc cttgccttaa taaatatgta atataaattt aagcaaacgt 2820
ctattttgta tatttgtaaa ctacaaagta aaatgaacat tttgtggagt ttgtattttg 2880
catactcaag gtgagaatta agttttaaat aaacctataa tattttatct gaaaaaaaaa 2940
aaaaaaaaa 2949

<210> 10
<211> 3057
<212> DNA
<213> Mus musculus
<400> 10
agttccccgg ccaagagagc gagcgcggct ccgggcgcgc ggggagcaga ggcggtggcg 60
ggcggcggcg gcacccggag ccgccgagtg cccctccccg cccctccagc cccccaccca 120
gcaacccgcc cgtgacccgc gcccatggcc gcgcgcaccc ggcacagtcc ccaggactcc 180
gcaccccgcg ccaccgccca gctcgcagtt ccgcgccacc gcggccattc tcacctggcg 240
gcgccgcccg cccaccgccc ggaccacagc ccccgcgccg ccgacagcca cagtggccgc 300
gacaacggtg ggggacactg ctgagtccaa gagcgtgcag cctggccatc ggacctactt 360
atctgccttg ctgattgtct atttttataa gagtttacaa cttttctaag aatttttgta 420
tacaaaggaa cttttttaaa gacatcgccg gtttatattg aatccaaaga agaaggatct 480
cgggcaatct gggggttttg gtttgaggtt ttgtttctaa agtttttaat cttcgttgac 540
tttggggctc aggtacccct ctctcttctt cggactccgg aggaccttct gggcccccac 600
attaatgagg cagccacctg gcgagtctga catggctgtc agcgacgctc tgctcccgtc 660
cttctccacg ttcgcgtccg gcccggcggg aagggagaag acactgcgtc cagcaggtgc 720
cccgactaac cgttggcgtg aggaactctc tcacatgaag cgacttcccc cacttcccgg 780
ccgcccctac gacctggcgg cgacggtggc cacagacctg gagagtggcg gagctggtgc 840
agcttgcagc agtaacaacc cggccctcct agcccggagg gagaccgagg agttcaacga 900
cctcctggac ctagacttta tcctttccaa ctcgctaacc caccaggaat cggtggccgc 960
caccgtgacc acctcggcgt cagcttcatc ctcgtcttcc ccggcgagca gcggccctgc 1020
cagcgcgccc tccacctgca gcttcagcta tccgatccgg gccgggggtg acccgggcgt 1080
ggctgccagc aacacaggtg gagggctcct ctacagccga gaatctgcgc cacctcccac 1140
ggcccccttc aacctggcgg acatcaatga cgtgagcccc tcgggcggct tcgtggctga 1200
gctcctgcgg ccggagttgg acccagtata cattccgcca cagcagcctc agccgccagg 1260
tggcgggctg atgggcaagt ttgtgctgaa ggcgtctctg accacccctg gcagcgagta 1320
cagcagccct tcggtcatca gtgttagcaa aggaagccca gacggcagcc accccgtggt 1380
agtggcgccc tacagcggtg gcccgccgcg catgtgcccc aagattaagc aagaggcggt 1440
cccgtcctgc acggtcagcc ggtccctaga ggcccatttg agcgctggac cccagctcag 1500
caacggccac cggcccaaca cacacgactt ccccctgggg cggcagctcc ccaccaggac 1560
tacccctaca ctgagtcccg aggaactgct gaacagcagg gactgtcacc ctggcctgcc 1620
tcttccccca ggattccatc cccatccggg gcccaactac cctcctttcc tgccagacca 1680
gatgcagtca caagtcccct ctctccatta tcaagagctc atgccaccgg gttcctgcct 1740
gccagaggag cccaagccaa agaggggaag aaggtcgtgg ccccggaaaa gaacagccac 1800
ccacacttgt gactatgcag gctgtggcaa aacctatacc aagagttctc atctcaaggc 1860
acacctgcga actcacacag gcgagaaacc ttaccactgt gactgggacg gctgtgggtg 1920
gaaattcgcc cgctccgatg aactgaccag gcactaccgc aaacacacag ggcaccggcc 1980
ctttcagtgc cagaagtgtg acagggcctt ttccaggtcg gaccaccttg ccttacacat 2040
gaagaggcac ttttaaatcc cacgtagtgg atgtgaccca cactgccagg agagagagtt 2100
cagtattttt ttttctaacc tttcacactg tcttcccacg aggggaggag cccagctggc 2160
aagcgctaca atcatggtca agttcccagc aagtcagctt gtgaatggat aatcaggaga 2220
aaggaagagt tcaagagaca aaacagaaat actaaaaaca aacaaacaaa aaaacaaaca 2280
aaaaaaacaa gaaaaaaaaa tcacagaaca gatggggtct gatactggat ggatcttcta 2340
tcattccaat accaaatcca acttgaacat gcccggactt acaaaatgcc aaggggtgac 2400
tggaagtttg tggatatcag ggtatacact aaatcagtga gcttgggggg agggaagacc 2460
aggattccct tgaattgtgt ttcgatgatg caatacacac gtaaagatca ccttgtatgc 2520
tctttgcctt cttaaaaaaa aaaaaagcca ttattgtgtc ggaggaagag gaagcgattc 2580
aggtacagaa catgttctaa cagcctaaat gatggtgctt ggtgagtcgt ggttctaaag 2640
gtaccaaacg ggggagccaa agttctccaa ctgctgcata cttttgacaa ggaaaatcta 2700
gttttgtctt ccgatctaca ttgatgacct aagccaggta aataagcctg gtttatttct 2760
gtaacatttt tatgcagaca gtctgttatg cactgtggtt tcagatgtgc aataatttgt 2820
acaatggttt attcccaagt atgcctttaa gcagaacaaa tgtgtttttc tatatagttc 2880
cttgccttaa taaatatgta atataaattt aagcaaactt ctattttgta tatttgtaaa 2940
ctacaaagta aaaaaaaatg aacattttgt ggagtttgta ttttgcatac tcaaggtgag 3000
aaataagttt taaataaacc tataatattt tatctgaacg acaaaaaaaa aaaaaaa 3057

<210> 11
<211> 2393
<212> DNA
<213> Rattus norvegicus
<400> 11
atcttcgttg acttcggggt ttgggtaccc ctctctcttc ttcggactcc ggaggacctt 60

ctgggccccc acattaatga ggcagccacc tggcgagtct gacatggctg tcagcgacgc 120
tctgctcccg tccttctcca cgttcgcgtc cggcccggcg ggaagggaga agacactgcg 180
tccagcaggt gccccgacta accgttggcg agaggaactc tctcacatga agcgacttcc 240
cccacttccc ggccgcccct acgacctggc ggcgacggtg gccacagacc tggaaagtgg 300
tggagctggt gcagcttgca gcagtaacaa cccggcccta ccccggaggg agaccgagga 360
gttcaacgat ctcctggacc tagactttat cctttccaac tcgctatccc accaggaatc 420
ggtggccgcc accgtgacca cctcggcgtc agcttcatcc tcgtcttccc cagctagcag 480
cggccctgcc agcgcgccct ccacctgcag cttcagctat ccgatccggg ccgggggtga 540
cccgggcgtg gctgcgggca acacaggtgg agggctcctc tacagccgag aatctgcgcc 600
acctcccacg gcccccttca acctggcgga catcaatgac gtgagcccct cgggcggctt 660
cgtggctgag ctcctgcggc cggagttgga cccagtatac attccgccac agcagcctca 720
gccgccaggt ggcgggctga tgggcaagtt tgtgctgaag gcgtctctga gcacccctgg 780
cagcgagtac accagccctt cggtcatcag tgttagcaaa ggaagcccag acggcagcca 840
ccctgtggta gtggcgccct acagcggtgg cccgccgcgt atgtgcccca agattaagca 900
agaggccgtc ccgtcctgca cggtcagccg gtccctagag gcccacttga gcgctggacc 960
ccagctcagc aacggccaca ggcccaacac acacgacttc cccctggggc ggcagctccc 1020
caccaggact acccctacac tgagtcccga ggaactgctg aacagcaggg actgtcaccc 1080
tggcctgcct cttcccccag gattccatcc ccatccgggg cccagctacc ctcctttcct 1140
gccagaccag atgcagtcgc aagtcccctc tctccattat caagagctca tgccaccggg 1200
atcctgcctg ccagaggagc ccaagccaaa gaggggaaga aggtcttggc cccggaaaag 1260
aacagccacc cacacttgtg actatgcagg ctgtggcaaa acctatacga agagttctca 1320
tctcaaggca cacctgcgaa ctcacacagg cgagaaacct taccactgtg actgggacgg 1380
ctgtgggtgg aaattcgccc gctcagatga actgaccagg cactaccgca aacacaccgg 1440
gcaccggccc tttcagtgcc agaagtgcga cagggccttt tccaggtcgg accaccttgc 1500
cttacacatg aagaggcact tttaaattcc acatcgtgga catgacccac actgccagga 1560
gagagttcag tatttttttt taacctttca cactgtcttc ccacgagggg aggagcccag 1620
ctggcaagcg ctacaatcat ggtcaagttc ccagcaagtc agcttgtgaa tggataatca 1680
ggagaaagga agagtccaag ggacaaaaga aaagaaaaga aaaaaatact aaaaaacaaa 1740
caaacaaaaa aaaaaaacaa aagaaaaaaa tcacagaaca gatggggtct gagactggat 1800
cttctatcat tccaatacca aatccgactt gaacaagact ggacttacaa aatgccaagg 1860
ggtgactgga agtttgtgga tatcagggta tacattaaat cagtgacctg gggggaggga 1920
agaccagagt tcccttgaat tgtgcttcaa tgatgcaata tacatggaaa gaccaccttg 1980
tatgctcttt gccttctaaa aagccattat gacgtcagag gaagaggaag caattcaggt 2040
acagaacgtg ttctaatagc ctaaacgatg gtgcttggtg agtcgtggtt ctaaaggtac 2100
caaacggggg agccaaagtt ctccaactgc tgcatacttt gacaaggaaa atctattttt 2160
gtcttccgat ctacatttat gacctaagtc aggtaaataa gcctggttta tttctgtaac 2220
attttttatg cagacagtct gttatgcact gtggtttcag atgtgcaata atttgtacaa 2280
tggtttattc ccaagtatgc ctttaagcag aacaaatgtg tttttctata tagttccttg 2340
ccttaataaa tatgtaatat aaatttaaaa aaaaaaaaaa aaaaaaaaaa aaa 2393

<210> 12
<211> 4018
<212> DNA
<213> Homo sapiens
<400> 12
   caggcagcgc tgcgtcctgc tgcgcacgtg ggaagccctg gccccggcca cccccgcgat 60
   gccgcgcgct ccccgctgcc gagccgtgcg ctccctgctg cgcagccact accgcgaggt 120
   gctgccgctg gccacgttcg tgcggcgcct ggggccccag ggctggcggc tggtgcagcg 180
   cggggacccg gcggctttcc gcgcgctggt ggcccagtgc ctggtgtgcg tgccctggga 240
   cgcacggccg ccccccgccg cccctccctt ccgccaggtg tcctgcctga aggagctggt 300
   ggcccgagtg ctgcagaggc tgtgcgagcg cggcgcgaag aacgtgctgg ccttcggctt 360
   cgcgctgctg gacggggccc gcgggggccc ccccgaggcc ttcaccacca gcgtgcgcag 420
   ctacctgccc aacacggtga ccgacgcact gcgggggagc ggggcgtggg ggctgctgct 480
   gcgccgcgtg ggcgacgacg tgctggttca cctgctggca cgctgcgcgc tctttgtgct 540
   ggtggctccc agctgcgcct accaggtgtg cgggccgccg ctgtaccagc tcggcgctgc 600
   cactcaggcc cggcccccgc cacacgctag tggaccccga aggcgtctgg gatgcgaacg 660
   ggcctggaac catagcgtca gggaggccgg ggtccccctg ggcctgccag ccccgggtgc 720
   gaggaggcgc gggggcagtg ccagccgaag tctgccgttg cccaagaggc ccaggcgtgg 780
   cgctgcccct gagccggagc ggacgcccgt tgggcagggg tcctgggccc acccgggcag 840
   gacgcgtgga ccgagtgacc gtggtttctg tgtggtgtca cctgccagac ccgccgaaga 900
   agccacctct ttggagggtg cgctctctgg cacgcgccac tcccacccat ccgtgggccg 960
   ccagcaccac gcgggccccc catccacatc gcggccacca cgtccctggg acacgccttg 1020
   tcccccggtg tacgccgaga ccaagcactt cctctactcc tcaggcgaca aggagcagct 1080
   gcggccctcc ttcctactca gctctctgag gcccagcctg actggcgctc ggaggctcgt 1140
   ggagaccatc tttctgggtt ccaggccctg gatgccaggg actccccgca ggttgccccg 1200
   cctgccccag cgctactggc aaatgcggcc cctgtttctg gagctgcttg ggaaccacgc 1260
   gcagtgcccc tacggggtgc tcctcaagac gcactgcccg ctgcgagctg cggtcacccc 1320
   agcagccggt gtctgtgccc gggagaagcc ccagggctct gtggcggccc ccgaggagga 1380
   ggacacagac ccccgtcgcc tggtgcagct gctccgccag cacagcagcc cctggcaggt 1440
   gtacggcttc gtgcgggcct gcctgcgccg gctggtgccc ccaggcctct ggggctccag 1500
   gcacaacgaa cgccgcttcc tcaggaacac caagaagttc atctccctgg ggaagcatgc 1560
   caagctctcg ctgcaggagc tgacgtggaa gatgagcgtg cgggactgcg cttggctgcg 1620
   caggagccca ggggttggct gtgttccggc cgcagagcac cgtctgcgtg aggagatcct 1680
   ggccaagttc ctgcactggc tgatgagtgt gtacgtcgtc gagctgctca ggtctttctt 1740
   ttatgtcacg gagaccacgt ttcaaaagaa caggctcttt ttctaccgga agagtgtctg 1800
   gagcaagttg caaagcattg gaatcagaca gcacttgaag agggtgcagc tgcgggagct 1860
   gtcggaagca gaggtcaggc agcatcggga agccaggccc gccctgctga cgtccagact 1920
   ccgcttcatc cccaagcctg acgggctgcg gccgattgtg aacatggact acgtcgtggg 1980
   agccagaacg ttccgcagag aaaagagggc cgagcgtctc acctcgaggg tgaaggcact 2040
   gttcagcgtg ctcaactacg agcgggcgcg gcgccccggc ctcctgggcg cctctgtgct 2100
   gggcctggac gatatccaca gggcctggcg caccttcgtg ctgcgtgtgc gggcccagga 2160
   cccgccgcct gagctgtact ttgtcaaggt ggatgtgacg ggcgcgtacg acaccatccc 2220
   ccaggacagg ctcacggagg tcatcgccag catcatcaaa ccccagaaca cgtactgcgt 2280
   gcgtcggtat gccgtggtcc agaaggccgc ccatgggcac gtccgcaagg ccttcaagag 2340
   ccacgtctct accttgacag acctccagcc gtacatgcga cagttcgtgg ctcacctgca 2400
   ggagaccagc ccgctgaggg atgccgtcgt catcgagcag agctcctccc tgaatgaggc 2460
   cagcagtggc ctcttcgacg tcttcctacg cttcatgtgc caccacgccg tgcgcatcag 2520
   gggcaagtcc tacgtccagt gccaggggat cccgcagggc tccatcctct ccacgctgct 2580
   ctgcagcctg tgctacggcg acatggagaa caagctgttt gcggggattc ggcgggacgg 2640
   gctgctcctg cgtttggtgg atgatttctt gttggtgaca cctcacctca cccacgcgaa 2700
   aaccttcctc aggaccctgg tccgaggtgt ccctgagtat ggctgcgtgg tgaacttgcg 2760
   gaagacagtg gtgaacttcc ctgtagaaga cgaggccctg ggtggcacgg cttttgttca 2820
   gatgccggcc cacggcctat tcccctggtg cggcctgctg ctggataccc ggaccctgga 2880
   ggtgcagagc gactactcca gctatgcccg gacctccatc agagccagtc tcaccttcaa 2940
   ccgcggcttc aaggctggga ggaacatgcg tcgcaaactc tttggggtct tgcggctgaa 3000
   gtgtcacagc ctgtttctgg atttgcaggt gaacagcctc cagacggtgt gcaccaacat 3060
   ctacaagatc ctcctgctgc aggcgtacag gtttcacgca tgtgtgctgc agctcccatt 3120
   tcatcagcaa gtttggaaga accccacatt tttcctgcgc gtcatctctg acacggcctc 3180
   cctctgctac tccatcctga aagccaagaa cgcagggatg tcgctggggg ccaagggcgc 3240
   cgccggccct ctgccctccg aggccgtgca gtggctgtgc caccaagcat tcctgctcaa 3300
   gctgactcga caccgtgtca cctacgtgcc actcctgggg tcactcagga cagcccagac 3360
   gcagctgagt cggaagctcc cggggacgac gctgactgcc ctggaggccg cagccaaccc 3420
   ggcactgccc tcagacttca agaccatcct ggactgatgg ccacccgccc acagccaggc 3480
   cgagagcaga caccagcagc cctgtcacgc cgggctctac gtcccaggga gggaggggcg 3540
   gcccacaccc aggcccgcac cgctgggagt ctgaggcctg agtgagtgtt tggccgaggc 3600
   ctgcatgtcc ggctgaaggc tgagtgtccg gctgaggcct gagcgagtgt ccagccaagg 3660
   gctgagtgtc cagcacacct gccgtcttca cttccccaca ggctggcgct cggctccacc 3720
   ccagggccag cttttcctca ccaggagccc ggcttccact ccccacatag gaatagtcca 3780
   tccccagatt cgccattgtt cacccctcgc cctgccctcc tttgccttcc acccccacca 3840
   tccaggtgga gaccctgaga aggaccctgg gagctctggg aatttggagt gaccaaaggt 3900
   gtgccctgta cacaggcgag gaccctgcac ctggatgggg gtccctgtgg gtcaaattgg 3960
   ggggaggtgc tgtgggagta aaatactgaa tatatgagtt tttcagtttt gaaaaaaa 4018

<210> 13
<211> 4237
<212> DNA
<213> Mus musculus
<400> 13
ggttcccgca cgtgggaggc ccatcccggc cttgagcaca atgacccgcg ctcctcgttg 60
ccccgcggtg cgctctctgc tgcgcagccg ataccgggag gtgtggccgc tggcaacctt 120
tgtgcggcgc ctggggcccg agggcaggcg gcttgtgcaa cccggggacc cgaagatcta 180
ccgcactttg gttgcccaat gcctagtgtg catgcactgg ggctcacagc ctccacctgc 240
cgacctttcc ttccaccagg tgtcatccct gaaagagctg gtggccaggg ttgtgcagag 300
actctgcgag cgcaacgaga gaaacgtgct ggcttttggc tttgagctgc ttaacgaggc 360
cagaggcggg cctcccatgg ccttcactag tagcgtgcgt agctacttgc ccaacactgt 420
tattgagacc ctgcgtgtca gtggtgcatg gatgctactg ttgagccgag tgggcgacga 480

cctgctggtc tacctgctgg cacactgtgc tctttatctt ctggtgcccc ccagctgtgc 540
ctaccaggtg tgtgggtctc ccctgtacca aatttgtgcc accacggata tctggccctc 600
tgtgtccgct agttacaggc ccacccgacc cgtgggcagg aatttcacta accttaggtt 660
cttacaacag atcaagagca gtagtcgcca ggaagcaccg aaacccctgg ccttgccatc 720
tcgaggtaca aagaggcatc tgagtctcac cagtacaagt gtgccttcag ctaagaaggc 780
cagatgctat cctgtcccga gagtggagga gggaccccac aggcaggtgc taccaacccc 840
atcaggcaaa tcatgggtgc caagtcctgc tcggtccccc gaggtgccta ctgcagagaa 900
agatttgtct tctaaaggaa aggtgtctga cctgagtctc tctgggtcgg tgtgctgtaa 960
acacaagccc agctccacat ctctgctgtc accaccccgc caaaatgcct ttcagctcag 1020
gccatttatt gagaccagac atttccttta ctccagggga gatggccaag agcgtctaaa 1080
cccctcattc ctactcagca acctccagcc taacttgact ggggccagga gactggtgga 1140
gatcatcttt ctgggctcaa ggcctaggac atcaggacca ctctgcagga cacaccgtct 1200
atcgcgtcga tactggcaga tgcggcccct gttccaacag ctgctggtga accatgcaga 1260
gtgccaatat gtcagactcc tcaggtcaca ttgcaggttt cgaacagcaa accaacaggt 1320
gacagatgcc ttgaacacca gcccaccgca cctcatggat ttgctccgcc tgcacagcag 1380
tccctggcag gtatatggtt ttcttcgggc ctgtctctgc aaggtggtgt ctgctagtct 1440
ctggggtacc aggcacaatg agcgccgctt ctttaagaac ttaaagaagt tcatctcgtt 1500
ggggaaatac ggcaagctat cactgcagga actgatgtgg aagatgaaag tagaggattg 1560
ccactggctc cgcagcagcc cggggaagga ccgtgtcccc gctgcagagc accgtctgag 1620
ggagaggatc ctggctacgt tcctgttctg gctgatggac acatacgtgg tacagctgct 1680
taggtcattc ttttacatca cagagagcac attccagaag aacaggctct tcttctaccg 1740
taagagtgtg tggagcaagc tgcagagcat tggagtcagg caacaccttg agagagtgcg 1800
gctacgggag ctgtcacaag aggaggtcag gcatcaccag gacacctggc tagccatgcc 1860
catctgcaga ctgcgcttca tccccaagcc caacggcctg cggcccattg tgaacatgag 1920
ttatagcatg ggtaccagag ctttgggcag aaggaagcag gcccagcatt tcacccagcg 1980
tctcaagact ctcttcagca tgctcaacta tgagcggaca aaacatcctc accttatggg 2040
gtcttctgta ctgggtatga atgacatcta caggacctgg cgggcctttg tgctgcgtgt 2100
gcgtgctctg gaccagacac ccaggatgta ctttgttaag gcagatgtga ccggggccta 2160
tgatgccatc ccccagggta agctggtgga ggttgttgcc aatatgatca ggcactcgga 2220
gagcacgtac tgtatccgcc agtatgcagt ggtccggaga gatagccaag gccaagtcca 2280
caagtccttt aggagacagg tcaccaccct ctctgacctc cagccataca tgggccagtt 2340
ccttaagcat ctgcaggatt cagatgccag tgcactgagg aactccgttg tcatcgagca 2400
gagcatctct atgaatgaga gcagcagcag cctgtttgac ttcttcctgc acttcctgcg 2460
tcacagtgtc gtaaagattg gtgacaggtg ctatacgcag tgccagggca tcccccaggg 2520
ctccagccta tccaccctgc tctgcagtct gtgtttcgga gacatggaga acaagctgtt 2580
tgctgaggtg cagcgggatg ggttgctttt acgttttgtt gatgactttc tgttggtgac 2640
gcctcacttg gaccaagcaa aaaccttcct cagcaccctg gtccatggcg ttcctgagta 2700
tgggtgcatg ataaacttgc agaagacagt ggtgaacttc cctgtggagc ctggtaccct 2760
gggtggtgca gctccatacc agctgcctgc tcactgcctg tttccctggt gtggcttgct 2820
gctggacact cagactttgg aggtgttctg tgactactca ggttatgccc agacctcaat 2880
taagacgagc ctcaccttcc agagtgtctt caaagctggg aagaccatgc ggaacaagct 2940
cctgtcggtc ttgcggttga agtgtcacgg tctatttcta gacttgcagg tgaacagcct 3000
ccagacagtc tgcatcaata tatacaagat cttcctgctt caggcctaca ggttccatgc 3060
atgtgtgatt cagcttccct ttgaccagcg tgttaggaag aacctcacat tctttctggg 3120
catcatctcc agccaagcat cctgctgcta tgctatcctg aaggtcaaga atccaggaat 3180
gacactaaag gcctctggct cctttcctcc tgaagccgca cattggctct gctaccaggc 3240
cttcctgctc aagctggctg ctcattctgt catctacaaa tgtctcctgg gacctctgag 3300
gacagcccaa aaactgctgt gccggaagct cccagaggcg acaatgacca tccttaaagc 3360
tgcagctgac ccagccctaa gcacagactt tcagaccatt ttggactaac cctgtctcct 3420
tccgctagat gaacatgggc attgtagcct cagcactcct ggatccacgt cacaagaggg 3480
actggtcagt tgtgaggcta ggtcatcctc caaacctctg tgtcatgggt ggtatgggag 3540
attgtcccag tgccttgttt cctgtaacag gcttgatttc tttcctgatg ccctcaggga 3600
ggcagatcct atccctttta gtggcaggga tccactagca ccagcacatg aggagtgcac 3660
ccagtgcaca tgggcactgg gacagtggac aggtgtgaga ttcctgggcc ctggagtctt 3720
ttcacaccta accatggagc ctgtcccagt acatcagagt gcctcggaga tgaaaaagga 3780
catcgagcca gtgacctaaa ttacagcctg aatatactct gaattcatgt gactgcctta 3840
gctacttctc tactgctgtg tagtaaaaca ccaagccaac ttataaaagc aggattttcc 3900
tactggagca gcagctgaga gtttacatct tgatccataa gcacaaaagc acaagacaga 3960
gagagagaga gagagagaga gagagagaga gagagagaga gagagagaga gagagagtca 4020
gtcagtcagt ctaacaaata actaagaaag gtgaagggtg atgaagtcca cagggatcac 4080
gctagggatg ttccatgcct tctctgaagc taagattcct tggcagcgtt tgacagtaac 4140
catagtgggt acctactgag atcactataa agataaaata gggggaagcg tatttgtact 4200
gaactggaaa aacatacaaa taaagagtaa atcatgg 4237

<210> 14
<211> 3378
<212> DNA
<213> Rattus norvegicus
<400> 14
atgccccgcg ctcctcgttg ccccgccgtg cgctctctac tgcgcagccg atatcgggag 60
gtgtggccgc tggcgacctt tgtgcggcgc ctggggcttg agggcagtcg gcttgtgcaa 120
cccggggacc cgaaggtctt ccgcacgttg gttgcccagt gcctagtgtg cgtgccctgg 180
ggctcacagc cgccacctgc tgacctttcc ttccaccagg tgtcatccct gaaagagctg 240
gtgtccaggg ttgtgcagaa actttgcgag cgcggtgaga ggaatgtgct ggcttttggc 300
tttgcactgc ttaacggggc cagaggtggg cctcccatgg ccttcacgac cagcgtgcat 360
agctacttgc ccaactcggt tactgagtcc ctgtgtgtca gtggtgcatg gatgctactg 420
ttgagccgag tgggcgacga cctgctggtc tacctgctgt cgcactgtgc gctctacctg 480
ctggtgcccc ccagctgtgc ctaccaggtg tgcgggtcac ccctgtacca aatttgtgcc 540
accacggata cctggtcctc tgtgcccgct ggttacaggc ccactcgacc cgtgggcggg 600
aatttcacta accttgggtc cgcacaccag atcaaaaaca gtggtcacca ggaagcacca 660
aaaccccagg ccttgccatc acgaggtacg aagaggcttc tgagtctcac cagtacaaac 720
gtgccttcag ctaagaaggc caggtttgaa cctgccctga gagtggataa gggaccccac 780
aggcaggtgg taccaacccc atcaggcaaa acatggcgcc caagtcctgc tgcgtccccc 840
aaggtgcctc ctgcagcgaa aaacttgtct ttgaaaggaa aggcatctga cccgagtctc 900
tctgggtcgg tgtgctgtaa acacaagccc agctcctcgt ccctgctgtc atcaccaccc 960
caagatgctg aaaagctcag gccattcact gagaccagac atttccttta ctccagggga 1020
ggtggccaag aggagctaaa tccctcattc ctactcaaca gcctcccgcc tagcttgacc 1080
ggggccagga gactggtgga gatcatcttt ctgggctcaa ggcctaggac atcaggacca 1140
ttctgcagga cccgccgcct gccccgtcga tactggcaga tgcgacccct attccagcag 1200
ctgctcatga accacgcaaa gtgccaatat gtcagattcc tccggtcgca ctgcagattt 1260
cgaacagcaa accagcgggt gccggatgcc atggacacca gcccatccca cctcacgagt 1320
ttgctccggt tacacagcag cccctggcag gtatacggct ttcttcgggc ctgcctccgc 1380
gagctggtgc ctgccggtct ctggggcacc aggcacaatg agcgccgctt cttaaagaac 1440
gtgaagaagt tcatctcgtt ggggaagtac gccaagctat ccctgcagga actgatgtgg 1500
agggtgaaag tggaggactg ccactggctc cgcagcagcc cagagaagga cactgtccct 1560
gccgcagagc accgtctgag ggagaggatc cttgccatgt tcctgttctg gctaatggac 1620
acatatgtgg tacagctgct gaggtcattc ttctacatca cagagaccac gttccagaag 1680
aaccgccttt tcttctaccg taagagtgtg tggagcaagc tgcagagcat tggaatcagg 1740
caacagcttg agagagttca gctacgggaa ctgtcacaag aggaggtcaa gcatcaccag 1800
gacacttggc tggccatgcc tatctgcaga ttgcgcttca tccccaagct caatggtctc 1860
cggcccattg tgaacatgag ttatggcatg gacaccagag cttttggcaa aaagaagcag 1920
acccagtgtt tcactcagag tctcaagact ttgttcagcg tgctcaacta cgagcggacc 1980
aaacatccta accttatggg tgcttcagta ctgggtacga gtgacagcta caggatctgg 2040
cggaccttcg tgctgcgtgt gcgtgctctg gaccagacac ccaggatgta ctttgttaag 2100
gcagatgtga caggggccta tgatgccatc ccccaggaca agctcgtgga aattgtcgcc 2160
aatataatca ggcgctcaga gagcatgtac tgtatccgcc agtatgcagt ggttcagaaa 2220
gatagccaag gccaagtcca caagtccttc aggagacagg tctccaccct ctctgacctc 2280
cagccataca tgggccagtt caccaagcat ctgcaggact cagatgccag tgcactgagg 2340
aactctgttg tcatcgagca gagcatctcc atgaatgaga ctggcagtag cctgctccac 2400
ttcttcctgc gctttgtccg tcacagtgtc gtgaagatcg atggcaggtt ctatgtgcaa 2460
tgccagggca tcccccaggg ctccagcctg tccaccctgc tctgcagtct gtgtttcgga 2520
gacatggaga acaagctgtt tgccgaggtg cagcaggacg gcttgctttt acgttttgtc 2580
gatgactttc tgttggtgac acctcacctg gcccatgcaa aagcctttct cagcaccctg 2640
gtccatggcg tgcccgagta tggctgcatg ataaacttgc agaagacagt ggtgaacttc 2700
cctgtggaga ccggcgccct gggaggtgca gccccgcacc agctgcctgc tcactgcctg 2760
tttccctggt gtggcttact gctggacact cggactttgg aagtattctg tgactactca 2820
ggttacggac ggacctcaat taagatgagc ctcaccttcc agggtgtctc cagggccggg 2880
aagaccatgc ggtacaagct cttgtcagtc ttgcggttga agtgtcatgg tctgtttcta 2940
gacttgcagg tgaacagcct gcagacagtc tgcatcaata tatacaagat cttcctgctt 3000
caggcctaca ggttccatgc atgtgtgatt cggcttccct ttggccagca tgttaggaag 3060
aaccatgcat tctttctggg catcatctcc aacctagcat cctgctgcta cgccatcctg 3120
aaggtcaaga atccaggagt gtcactaagg gccaagggtg cccctggctc ctttccgccc 3180
gaggccacac gttggctctg ctaccaagcc ttcctgctca agctggctgc tcattctgtc 3240
acctacaagt gtctcctggg acctcttagg acagcccaaa aacagctgtg ccggaagctc 3300
ccagaggcaa caatgaccct ccttaagact gcagctgacc cagccctaag cacagatttt 3360
cagaccattt tggactaa 3378

<210> 15
<211> 1903
<212> DNA
<213> Homo sapiens
<400> 15
cggcgtcccc ggggcccact cccgagcgca ggcgggcagc caggcgggcg gcgcggcgcg 60
ggccggcagg aagcgtattc tgggcacggg gcgccgggcg ggccggctgc gccgagcggc 120
agtggtggga taccacccaa ggcctcgcgc ggcgccgccc gtcgaggggc gggcggcggc 180
gtagccactg ggccgtcgaa gagcgcagga ggccggtggg ccgggccggg ccgcgcggcg 240
cagccatgcc tggctttacg tgctgcgtgc caggctgcta caacaactcg caccgggaca 300
aggcgctgca cttctacacg tttccaaagg acgctgagtt gcggcgcctc tggctcaaga 360
acgtgtcgcg tgccggcgtc agtgggtgct tctccacctt ccagcccacc acaggccacc 420
gtctctgcag cgttcacttc cagggcggcc gcaagaccta cacggtacgc gtccccacca 480
tcttcccgct gcgcggcgtc aatgagcgca aagtagcgcg cagacccgct ggggccgcgg 540
ccgcccgccg caggcagcag cagcaacagc agcagcagca gcaacagcag caacagcagc 600
agcagcagca acagcagcag cagcagcagc agcagcagca gtcctcaccc tctgcctcca 660
ctgcccagac tgcccagctg cagccgaacc tggtatctgc ttccgcggcc gtgcttctca 720
cccttcaggc cactgtagac agcagtcagg ctccgggatc cgtacagccg gcgcccatca 780
ctcccactgg agaagacgtg aagcccatcg atctcacagt gcaagtggag tttgcagccg 840
cagagggcgc agccgctgcg gccgccgcgt cggagttaca ggctgctacc gcagggctgg 900
aggctgccga gtgccctatg ggcccccagt tggtggtggt aggggaagag ggcttccctg 960
atactggctc cgaccattcg tactccttgt cgtcaggcac cacggaggag gagctcctgc 1020
gcaagctgaa tgagcagcgg gacatcctgg ctctgatgga agtgaagatg aaagagatga 1080
aaggcagcat tcgccacctg cgtctcactg aggccaagct gcgcgaagaa ctgcgtgaga 1140
aggatcggct gcttgccatg gctgtcatcc gcaagaagca cggaatgtga actggtgccc 1200
cggcagcctg ctggactccc agaccccatc cagccagggg accgcaggcc attgttgaac 1260
tcctctatac tcctgggcac tggttgacag tactgaggct taaggcagct ggactctctt 1320
gctggtgacc tggcatcctc aattgtttcc tcctgaagtg gaagctgggg ccttagactc 1380
tgccctggtg acaccagcaa ttatgacttt gtctaccctt cctccccagt tattgttgca 1440
gattctggtt aagcagaggc ttcagaacca ctgaacttga aacttaccct ctagggatgc 1500
aggtgggatg tccagggact ataggtttgg gaaaaccata ccttaaggtt ggtcagcagt 1560
cagacaactc taatgtgtgt agtgataaga gattcaagta acatcagttc tcctcctttt 1620
catgcttttc cttcccaggt gcagcctgtg attctgatgg ggactggtaa atctgtgcct 1680
ctgcctccta ggacttattt tcccaggagg ccatttacaa ggggatctgg atgacctgct 1740
gatggagatc cagcttgcca gggacttagg tttatcctgt tttgtttgct actggttaca 1800
aattctattt tctgtacaat tagtcagact aaagttttca ctgtgtttgt ttggcaaaac 1860
aaattaaaca aaaagtaagg tttttaaaaa aaaaaaaaaa aaa 1903

<210> 16
<211> 1832
<212> DNA
<213> Mus musculus
<400> 16
gaagcgcagg cggacggccg ggtgggtagc agcggcgcgg gcggtccgca accatattct 60
gggcacgggg cctcggcccg ggacgactgc gccgggcggc aatagtgaga ggcctctgag 120
agcctcgcgc ggcgccgccc gtcgaggagc tgagacaggg gccagacccg gccgtcgaag 180
agctcgagag gccggtgggc cgggccgcgc gtcgcagcca tgcctggctt tacgtgctgc 240
gttccgggct gctacaacaa ttcacaccgg gacaaggcgc tgcacttcta cacgtttccc 300
aaggacgctg agttgcggcg cctctggctc aagaacgtgt cccgtgctgg cgtcagtggg 360
tgcttctcca ccttccaacc caccaccggc caccgtctct gcagcgtcca ctttcagggc 420
ggccgcaaga cctacacggt gcgcgttccc accattttcc cgctgcgtgg cgtcaatgag 480
cgcaaagtag ctcggagacc tgcgggagct gcggcagccc gccgtaggca gcagcagcag 540
caacaacaac agcagcagca gcaacagcag cagctgcaac agcagcagcc gtctccgtcc 600
tcctccactg cccagaccac ccagttgcag ccgaacctgg tgtctgcctc tgcagctgtg 660
cttcttacgc ttcaggccgc cgtagacagc aaccaggctc cgggatccgt ggttcccgtg 720
tccacgactc cctcgggaga tgatgtgaag cccatcgacc tcacagtgca agtcgagttt 780
gcagctgctg aaggggcagc cgccgctgcc gccgcctcag agctagaggc tgctacggct 840
gggctggagg ccgctgagtg cactctgggc cctcagctgg tggtggtagg ggaagagggc 900
ttccctgaca ctggctctga ccactcgtac tccttgtcct cgggtaccac ggaggaggag 960
ctcctgcgca agctgaacga gcagcgggac atcttggccc tgatggaagt gaagatgaag 1020
gagatgaagg gtagcatccg ccatctgcgt ctcaccgagg ccaagctccg tgaagaactt 1080
cgagagaagg atcgtctgct tgccatggct gtcatccgta agaagcacgg catgtgaatg 1140

gttcccccca gaaacctgca gaattcgtga ctccttccag cccaaggaat cctcaggcaa 1200
atgttgtact ccccagtatt cctgttgaca gtgccgaggt ttaggacagc gggactccag 1260
ttggtcagtt ggcaccttct ttcgtctcct cgtgaagtaa gagttgggga cttcagaatc 1320
tggtgacacc agcagttatg actttgtctc tcactcccca gtttatgttg cagattctgg 1380
ttatacacag gcttcagaac cactgaactt ggaacttacc ctggaggggg tgcagatgga 1440
actcttaagg gactgtgggt ttgaggaaac cacttcttca ggttggccag caatcagaga 1500
cagctttgct gtgtgtagtg ataagagatc tcagtaacat cgtttctcct ttccatgcta 1560
cccggttcag gtgcagtctg tgattctgat ggggactagc tgatctgtgc ctctgtctct 1620
taggacctct ctacccagaa ggtcatttat gagagggctc tgggtgactt gctgatggag 1680
atccagctca ccagggactt aggtttatct cgttatgttt gctactggtt acaaattcta 1740
ttttctgtac aattagactg aagttttcac tgtttggcaa aacaaattaa acaaaaaagt 1800
aaggttttta aaaaaaaaaa aaaaaggcca ca 1832

<210> 17
<211> 55
<212> DNA
<213> Escherichia coli
<400> 17
tctagagcgg ccgcccggcg ggtcgccacc atgagtgtgg atccagcttg tcccc 55

<210> 18
<211> 36
<212> DNA
<213> Escherichia coli
<400> 18
catgcaacct gaagacgtgt gaagactagt atcgat 36

<210> 19
<211> 51
<212> DNA
<213> Escherichia coli
<400> 19
tctagagcgg ccgcccggcg ggtcgccacc atggctgtca gcgacgcgct g 51

<210> 20
<211> 46
<212> DNA
<213> Escherichia coli
<400> 20
ccacctcgcc ttacacatga agaggcattt ttaaaagctt atcgat 46

<210> 21
<211> 50
<212> DNA
<213> Escherichia coli
<400> 21
tctagaggat ccccggcggg tcgccaccat ggcggccgcg ggaattcgat 50

<210> 22
<211> 39
<212> DNA
<213> Escherichia coli
<400> 22
ggcacactgc ccctctcaca catgtgaaag cttatcgat 39

<210> 23
<211> 51
<212> DNA
<213> Escherichia coli
<400> 23
tctagagcgg ccgcccggcg ggtcgccacc atggcgggac acctggcttc g 51

<210> 24

<211> 37
<212> DNA
<213> Escherichia coli
<400> 24
gggctctccc atgcattcaa actgaggatc catcgat                                37

<210> 25
<211> 50
<212> DNA
<213> Escherichia coli
<400> 25
tctagagcgg ccgcccggcg ggtcgccacc atgccgcgcg ctccccgctg                  50

<210> 26
<211> 41
<212> DNA
<213> Escherichia coli
<400> 26
caaaatccca agcgtggcgc tgcccttaaa agcttatcga t                           41

<210> 27
<211> 43
<212> DNA
<213> Escherichia coli
<400> 27
tctagagcgg ccgcccggcg ggtcgccacc atggtgagca agg                         43

<210> 28
<211> 38
<212> DNA
<213> Escherichia coli
<400> 28
ctcggcatgg acgagctgta caataaaagc ttatcgat                               38

## Description

The present invention relates to a process for preparing reprogrammed cells, in particular for preparing pluripotent and multipotent stem cells and to the pluripotent and multipotent stem cells prepared by said processes.

Aging - whether in vivo during the life course or in vitro during scale up - affects cells, in particular stem cells. For example, aged cells show increased apoptosis rates, decreased differentiation and proliferation capacity, and decreased therapeutic effectiveness. During aging, stem cells often differentiate into a less pliable, less proliferative form. Cell aging represents a problem in particular when using cells from elderly patients/donors as starting material for cell cultures, when expanding cells in vitro and when using cells that have a limited differentiation capacity for therapy or research.

One approach to dedifferentiate aged cells is to create so called Induced Pluripotent Stem Cells (IPS). The current state of the art foresees to use a viral transfer of transcription factors to induce increased differentiation potency in cells and to rejuvenate them. This, however, is potentially dangerous because a viral transfer permanently changes the genome of the cell and also might lead to complications because of the viral parts left behind in the cell genome. Moreover, viral integration into the genome is known to increase cancer risk in cells. Essentially the same applies to the use of plasmids for reprogramming cells (Okita et al., 2008, Science. 2008 Nov 7;322(5903):949-53. Generation of mouse induced pluripotent stem cells without viral vectors).

Another approach employs contacting cells to be reprogrammed with extracts of human embryonic stem cells for reprogramming cells. This approach, however, is ethically and practically problematic.

Some other approaches use chemical factors to dedifferentiate cells (e. g. US 200710254884 A1, US 200710020759 A1), but these factors are not efficacious in fully reprogramming cells to a pluripotent state by themselves.

Thus, the technical problem underlying the present invention is to provide improved methods to reprogram cells, in particular to dedifferentiate cells, which overcome the above-identified disadvantages, in particular allow in an easy, efficient and safe way the provision of reprogrammed, in particular dedifferentiated cells, preferably displaying multipotent, most preferably pluripotent, stem cell character.

The present invention solves its technical problem by the provision of a process for preparing reprogrammed cells in vitro, comprising the process steps, preferably in the given order, a) providing cells to be reprogrammed; b) introducing, preferably transfecting, mRNA molecules capable of reprogramming the cells into the cells provided in step a), wherein the reprogramming mRNA molecules are coding for at least one protein selected from the group consisting of: Ronin, Oct4, Klf4, Sox2, Nanog and TERT; and c) culturing the cells obtained in step b) in a cell culture medium and under a condition suitable to allow translation of the introduced, preferably transfected reprogramming mRNA molecules so as to obtain reprogrammed cells.

Thus, the present invention foresees a process which is able to prepare reprogrammed cells without the need to permanently change the genome of the cell and without the need to use viruses to transfer transcription factors or the like into the cell. The present invention also is advantageous in so far, as for reprogramming cells no extracts of human embryonic stem cells or cost intensive and potentially dangerous chemical factors are needed. Thus, ethical concerns, genetic stability problems and cancer risks are reduced or even avoided. In contrast, the present invention provides the advantageous teaching to introduce, preferably transfect, specific mRNA molecules, in the following termed "reprogramming mRNA molecules", into the cells, wherein these reprogramming mRNA molecules are able to reprogram the mRNA recipient cells, preferably the cells being transfected therewith, into a dedifferentiated status. Advantageously, the mRNA molecules introduced, preferably transfected, according to the present invention will not integrate into the recipient's genome and therefore do not pose a risk of cancer or genetic instability. The invention therefore foresees a process for preparing one or more reprogrammed cells in vitro wherein said process does not include any cloning amplification or proliferation of the mRNA recipient cells. The present invention also foresees a process for preparing one or more reprogrammed cells in vivo, that is in a living organ, a living organism or animal, more particular a mammal.

In the context of the present invention the term "reprogramming" preferably means remodelling, in particular erasing and/or remodelling, epigenetic marks of a cell such as DNA methylation, histon methylation or activating genes by inducing transcription factor signal systems as for oct4. In particular, the reprogramming of the present invention provides at least one dedifferentiated and/or rejuvenated cell, in particular provides a cell having the characteristic of a multipotent, in particular pluripotent stem cell. Thus, in case the cell to be reprogrammed is cells which already have a multipotent or pluripotent character, the present invention is able to maintain these cells by the reprogramming of the present invention in their multi- or pluripotent state for a prolonged period of time. In case the cells to be reprogrammed are in an aged or differentiated state, the present invention allows the dedifferentiation into a multipotent or pluripotent stem cell. In a particularly preferred embodiment, multipotent cells may be reprogrammed to become pluripotent cells.

In particular, the term "reprogrammed cells" designates cells which have been changed to have a higher differentiation and/or proliferation potential. Also, in a preferred embodiment the reprogramming of asomaticdifferentiated cell toward a multipotent stem cell or "young" differentiated cell is termed reprogramming and the product is called a reprogrammed cell.

In particular, the present process allows it to enrich immature stem cells, preferably showing a high telomerase activity, several multipotent, preferably pluripotency, markers and an increased secretion of growth factors. The present invention provides the advantage that aged stem cells can be expanded for a longer period of time and with a higher stem cell yield. Further, stem cell lines and tissue engineered construct drafts made or derived from cells reprogrammed by the present process show a reduced minimal immunological risk, in particular if the patient owns cells are used as a source for the reprogramming. Thus, the present invention provides means and methods to prepare in particular pluripotent stem cells, to provide multipotent stem cells, to provide means and methods for an increased expansion of cells or stem cells in vitro and for the rejuvenation of aged cells or aged stem cells for tissue engineering or cell therapies.

In particular, the present invention foresees, preferably in a first step, to provide cells to be reprogrammed. Preferably, the cells to be reprogrammed may be adult, neonatal or embryonic differentiated cells, but are not limited thereto. Preferably, the cells to be reprogrammed are adult or neonatal undifferentiated cells. All of these cells may, in a preferred embodiment, be cell lines, immortalized cells, cells kept in cell culture, isolated cell populations, preferably cells isolated from a donor, either a living or dead donor, or cells isolated from the environment. Cell types which can be reprogrammed according to the present invention potentially are all cell types, and are in preferred instances fibroblasts, hepatocytes, cardiac cells, cardiomyocytes, nerve cells, chondrocytes, osteoblasts, adipocytes, myoblasts, hepatoblasts, hepatic stem cells, insulin producing cells, neural stem cells, cardiomyogenic cells, dermatocytes, keratinocytes, pancreatic cells, monocytes, epithelial cells or mesenchymal stem cells (MSC). Preferably, the cells may be mammalian cells, in particular human cells or animal cells, preferably ovine, horse, ape or in particular rodent cells, preferably hamster cells, mouse cells or rat cells. The cells may also be fish, reptile, avian, amphibian or insect cells. Preferably, the cells to be reprogrammed are lineage-committed cells. In a further preferred embodiment the cells, in particular the cells of the above identified origin, are stem cells, in particular post-natal stem cells or non-embryonic stem cells.

A suitable and preferred stem cell source like mesenchymal stem cells is a tissue within the human or animal body which comprises the stem cells for use in the present invention, optionally together with other cell types. Preferably, the suitable stem cell source is bone marrow, both adult and fetal, cytokine or chemotherapy mobilized peripheral blood, fetal liver, umbilical cord blood, embryonic yolk sac and spleen, both adult and fetal, more preferably the stem cell source is adult bone marrow or umbilical cord blood, most preferably the stem cell source is bone marrow. Bone marrow cells may be obtained from any known source, including, but not limited to, ilium, sternu, tibiae, femora, spine or other bone cavities. Preferably, the stem cells are isolated from a mammalian organism such as human, mouse or rat, and more preferably the stem cells are isolated from a human organism.

The term "isolated cell population" is intended to mean that the cells are not in contact with other cells with which they are usually in contact within the body of the mammal or in a tissue sample obtained directly, i.e. without purification or enrichment step, from the mammal. A "cell population" according to the present invention may comprise not only cells of one cell type such as fibroblasts or mesenchymal stem cells as defined for example by the expression of a specific combination of surface markers, but also a mixture of cells of different cell types which show different combinations of surface markers.

The cells harvested from said sources may be used directly for transfection or may be cryoconserved by freezing them at a temperature from about - 196 °C to about - 130°C.

The cells to be reprogrammed receive, according to the present invention, in a second step at least one species of reprogramming mRNA molecules, in particular linear and isolated mRNA molecules, selected from the group of mRNA molecules encoding Ronin, mRNA molecules encoding Oct4, mRNA molecules encoding Klf4, mRNA molecules encoding Sox2, mRNA molecules encoding Nanog and mRNA molecules encoding TERT. Ronin, Oct4, Klf4, Sox2, Nanog and TERT are proteins with the ability to reprogram cells. In the context of the present invention these proteins are termed "reprogramming proteins" and are characterised by their ability to reprogram target cells and therefore by their regulatory function in terms of determining the cell fate, in particular being able to dedifferentiate a target cell and/or maintain a cell in a dedifferentiated state, preferably by being transcription factors.

Differentiation behaviour of cells and state of differentiation can be detected according to the methods set forth below. However any method for determining the differentiation state or potency of a cell known the skilled person are applicable as well.

In a preferred embodiment, the cell to be reprogrammed is transfected with one or more reprogramming mRNA molecules according to the present invention. In another preferred embodiment the cell to be reprogrammed is supplemented with one or more reprogramming mRNA molecules according to the present invention. In another preferred embodiment the cell to be reprogrammed is injected with one or more reprogramming mRNA molecules according to the present invention.

The present invention thus foresees to use one or more of mRNA molecules encoding Ronin, Oct4, Klf4, Sox2, Nanog or TERT. These mRNA molecules may, in a preferred embodiment, be molecules having the wild-type mammalian, in particular human, animal, preferably rodent, more preferably mouse, hamster or rat or any other animal nucleotide sequence. In a preferred embodiment of the present invention the reprogramming mRNA molecule is selected from the group consisting of mRNA nucleotide sequence molecules being encoded by any one of the DNA sequences given under SEQ ID No. 1 to 16.

Of course, the invention also foresees to use mRNA molecules, whose sequence is a functional equivalent to the polynucleotide sequence given above. In the context of the present invention a functional equivalent is a nucleotide sequence molecule, which either codes with a different nucleotide sequence exactly the same protein as the mRNA sequence encoded by any one of SEQ ID No. 1 to 16 or is a mRNA sequence, which encodes a protein with a different amino acid sequence but with same or similar function, in particular being able to reprogram the cells according to the present invention.

In a particularly preferred embodiment of the present invention the functional equivalent of the mRNA molecules is a polynucleotide with a homologous sequence to the wild-type mRNA polynucleotide as encoded by the DNA sequence in any one of SEQ ID No. 1 to 16. In a preferred embodiment of the present invention the degree or percentage of homology is at least 50, 60, 70, 80, 90, 95, 99 or 100%. In a furthermore preferred embodiment a functional equivalent of the mRNA molecules as given in any one of SEQ ID No. 1 to 16 is a mRNA sequence which is encoded by a DNA sequence being substantially complementary to the sequences of any one of SEQ ID No. 1 to 16 or to its complementary, preferably substantially, complementary sequence. In a furthermore preferred embodiment the functional equivalent of the mRNA molecule as encoded by any one of the DNA sequences of SEQ ID No. 1 to 16 is a mRNA molecule being encoded by a DNA sequence which is able to hybridize under stringent or reduced stringent conditions to any one of the polynucleotides given in SEQ ID No. 1 to 16 or to its complementary, preferably substantially, complementary sequence.

In the context of the present invention the term "mRNA molecule" is meant to refer to a linear polymer of ribonucleotide molecules, which is single-stranged and serves as a template for protein synthesis.

Polynucleotides have "homologous" sequences if the sequence of nucleotides in the two sequences is the same when aligned for maximum correspondence as described herein. Sequence comparison between two or more polynucleotides is generally performed by comparing portions of the two sequences over a comparison window to identify and compare local regions of sequence similarity. The comparison window is generally from about 20 to 200 contiguous nucleotides.

The "percentage of sequence homology" for polynucleotides, herein referred to be 50, 60, 70, 80, 90, 95, 98, 99 or 100 percent sequence homology, may be determined by comparing two optimally aligned sequences over a comparison window, wherein the portion of the polynucleotide sequence in the comparison window may include additions or deletions (i.e. gaps) as compared to the reference sequence (which does not comprise additions or deletions) for optimal alignment of the two sequences. The percentage is calculated by: (a) determining the number of positions at which the identical nucleic acid base occurs in both sequences to yield the number of matched positions; (b) dividing the number of matched positions by the total number of positions in the window of comparison; and (c) multiplying the result by 100 to yield the percentage of sequence homology. Optimal alignment of sequences for comparison may be conducted by computerized implementations of known algorithms, or by inspection. Readily available sequence comparison and multiple sequence alignment algorithms are, respectively, the Basic Local Alignment Search Tool (BLAST) (Altschul, S.F. et al. 1990. J. Mol. Biol. 215:403; Altschul, S.F. et al. 1997. Nucleic Acid Res. 25:3389-3402) and ClustalW programs both available on the internet. Other suitable programs include GAP, BESTFIT and FASTA in the Wisconsin Genetics Software Package (Genetics Computer Group (GCG), Madison, WI, USA).

As used herein, "substantially complementary" means that two nucleic acid sequences in question have at least about 65%, preferably about 70%, more preferably about 80%, even more preferably 90%, and most preferably about 98%, sequence complementarity to each other. This means that the DNA sequence coding the functional equivalent and the polynucleotide given in any of SEQ ID No. 1 to 16 or its complement must in a preferred embodiment exhibit sufficient complementarity to hybridise under stringent conditions. A substantially complementary sequence is preferably one that has sufficient sequence complementarity to the nucleotide sequence in question to result in binding.

The term "primer" as used herein refers to an oligonucleotide which is capable of annealing to the amplification target allowing a DNA polymerase to attach thereby serving as a point of initiation of DNA synthesis when placed under conditions in which synthesis of primer extension product which is complementary to a nucleic acid strand is induced, i.e., in the presence of nucleotides and an agent for polymerization such as DNA polymerase and at a suitable temperature and pH. The (amplification) primer is preferably single stranded for maximum efficiency in amplification. Preferably, the primer is an oligodeoxy ribonucleotide. The primer must be sufficiently long to prime the synthesis of extension products in the presence of the agent for polymerization. The exact lengths of the primers will depend on many factors, including temperature and source of primer.

A "pair of bi-directional primers" as used herein refers to one forward and one reverse primer as commonly used in the art of DNA amplification such as in PCR amplification.

The terms "stringency" or "stringent hybridization conditions" refer to hybridization conditions that affect the stability of hybrids, e.g., temperature, salt concentration, pH, formamide concentration and the like. These conditions are empirically optimised to maximize specific binding and minimize nonspecific binding of an oligo- or polynucleotide to its target nucleic acid sequence. The terms as used include reference to conditions under which an oligo- or polynucleotide will hybridise to its target sequence, to a detectably greater degree than other sequences (e.g. at least 2-fold over background). Stringent conditions are sequence dependent and will be different in different circumstances. Longer sequences hybridise specifically at higher temperatures.

Generally, stringent conditions are selected to be about 5 DEG C (degree celcius) lower than the thermal melting point (Tm) for the specific sequence at a defined ionic strength and pH. The Tm is the temperature (under defined ionic strength and pH) at which 50% of a complementary target sequence hybridises to a perfectly matched oligo- or polynucleotide. Typically, stringent conditions will be those in which the salt concentration is less than about 1.0 M Na <+> ion, typically about 0.01 to 1.0 M Na <+> ion concentration (or other salts) at pH 7.0 to 8.3 and the temperature is at least about 30 DEG C for short probes or primers (e.g. 10 to 50 nucleotides) and at least about 60 DEG C for long probes or primers (e.g. greater than 50 nucleotides). Stringent conditions may also be achieved with the addition of destabilizing agents such as formamide.

Exemplary low stringent conditions or "conditions of reduced stringency" include hybridization with a buffer solution of 30% formamide, 1 M NaCl, 1% SDS at 37 DEG C and a wash in 2x SSC at 40 DEG C. Exemplary high stringency conditions include hybridization in 50% formamide, 1 M NaCl, 1% SDS at 37 DEG C, and a wash in 0.1x SSC at 60 DEG C. Hybridization procedures are well known in the art and are described in e.g. Ausubel et al, Current Protocols in Molecular Biology, John Wiley & Sons Inc., 1994.

Thus, the present invention foresees to introduce, preferably transfect, specific reprogramming mRNA molecules into the cells, which mRNA molecules once introduced, preferably transfected, in the cells are able to be translated into so called reprogramming proteins which can turn on specific genes in the cells, in particular multipotency and/or the pluripotency genes in the cell. The mRNA molecule used according to the present invention is preferably a linear molecule having a poly A tail, most preferably produced by in vitro transcription. Thus, in a preferred embodiment the mRNA molecules are produced by in vitro transcription, in particular using bacterial systems. In a particular preferred embodiment the DNA sequences of the reprogramming proteins are cloned into plasmids and amplified in bacteria, for instance E. coli. In a further preferred embodiment, the plasmids are then isolated from the bacteria, are linearised and subjected to restriction digestion. In a further preferred embodiment cDNA prepared by said method is transcribed into mRNA, which in turn is incubated to destroy cDNA residues and to obtain mRNA molecules to be introduced into the cells.

Since the introduced reprogramming mRNA molecules and the proteins translated therefrom are over the time being degraded in the cells, they cannot permanently integrate into the genome of the cell providing some of the advantageous features of the present invention.

In a preferred embodiment of the present invention the present teaching foresees to introduce, preferably transfect, the reprogramming mRNA molecules into the cell by electroporation, by lipofection, by injection, by magnetofection, by particle bombardment, gene gun, or by any other method known in the art suitable to introduce mRNA molecules into a target cell.

In a particular preferred embodiment the present invention further foresees in its step c) to culture the cells into which the reprogramming mRNA molecules have been introduced in a cell culture medium and under a condition suitable to allow translation of the transfecting reprogramming mRNA molecules, so as to obtain the reprogrammed cells.

In a particularly preferred embodiment of the present invention the present process therefore consists of the above-identified steps a), b) and c), preferably in this order. Accordingly, the present invention excludes any further process steps, in particular any further substantial process step, in particular any intervening or subsequent process steps. Thus, the present invention provides its advantages in a simple and cost-effective way.

In a particularly preferred embodiment the cell culture system is a cell culture system, comprising a cell culture medium, preferably in a culture vessel, in particular a cell culture medium supplemented with at least one so called "inducing substance", which is a substance suitable and determined for protecting the cells from in vitro aging and/or inducing in an unspecific or specific reprogramming. In a particularly preferred embodiment an inducing substance according to the present invention is a substance selected from the group consisting of reversin, resveratol, selenium, a selenium-containing compound, EGCG ((-)-epigallocatechin-3-gallate), valporic acid and salts of valporic adic, in particular sodium valporat.

In a particularly preferred embodiment the at least one inducing substance is present in the cell culture medium used in step c) of the present process in a concentration from 0.001 to 100 µM, preferably from 0.005 to 50 µM.

In a particularly preferred embodiment the present invention foresees to use a concentration of reversin from 0.5 to 10 µM, preferably of 1 µM. In a furthermore preferred embodiment the present invention foresees to use resveratrol in a concentration of 10 to 100 µM, preferably 50 µM. In a furthermore preferred embodiment the present invention foresees to use selenium or a selenium containing compound in a concentration from 0.05 to 0.5 µM, preferably of 0.1 µM. In a furthermore preferred embodiment the present invention foresees to use EGCG in a concentration from 0.001 to 0.1 µM, preferably of 0.01 µM. In a furthermore preferred embodiment the present invention foresees to use valpouric acid or sodium valporate in a concentration from 1 to 10 µM, in particular of 5 µM.

The present invention foresees in a furthermore preferred embodiment culturing the cells obtained in step b) in a cell culture medium, wherein the cell culture medium comprises, optionally in combination with the inducing substance as specified above, at least one transient proteolysis inhibitor. The use of at least one proteolysis inhibitor in the cell culture medium of the present invention increases the time the reprogramming proteins derived from the mRNA or any endogenous genes will be present in the cells and thus facilitates in an even more improved way the reprogramming by the transfected mRNA derived factors. The present invention uses in a particularly preferred embodiment as a transient proteolysis inhibitor a protease inhibitor, a proteasome inhibitor and/or a lysosome inhibitor. In a particularly preferred embodiment the proteosome inhibitor is selected from the group consisting of MG132, TMC-95A, TS-341 and MG262.

In a furthermore preferred embodiment the protease inhibitor is selected from the group consisting of aprotinin, G-64 and leupeptine-hemisulfat. In a furthermore preferred embodiment the lysosomal inhibitor is ammonium chloride.

In a furthermore preferred embodiment the present invention also foresees a cell culture medium comprising at least one transient inhibitor of mRNA degradation. The use of a transient inhibitor of mRNA degradation increases the half-life of the reprogramming factors as well.

In a furthermore preferred embodiment of the present invention a condition suitable to allow translation of the transfected reprogramming mRNA molecules in the cells is an oxygen content in the cell culture medium from 0.5 to 21 %, preferably from 1 to 20%, more preferably from 5 to 19% and particularly preferred from 10 to 18%. More particular, and without wishing to be bound to theory, oxygen is used to further induce or increase Oct4 by triggering Oct4 via Hif1a.

In a preferred embodiment conditions that are suitable to support reprogramming of the cells by the mRNA molecules in the cells are selected; more particularly, these conditions require a temperature from 30 to 38°C, preferably from 31 to 37°C, most preferably from 32 to 36°C.

The glucose content of the medium is in a preferred embodiment of the present invention below 4.6 g/l, preferably below 4.5 g/l, more preferably below 4 g/l, even more preferably below 3 g/l, particularly preferably below 2 g/l and most preferably it is 1 g/l. DMEM media containing 1 g/l glucose being preferred for the present invention are commercially available as "DMEM low glucose" from companies such as PAA, Omega Scientific, Perbio and Biosera. More particular, and without wishing to be bound to the theory, high glucose conditions adversely support aging of cells (methylation, epigenetics) in vitro which may render the reprogramming difficult.

In a furthermore preferred embodiment of the present invention the cell culture medium contains glucose in a concentration from 0.1 g/l to 4.6 g/l, preferably from 0.5 g/l to 4,5 g/l and most preferably from 1 g/l to 4 g/l.

The terms "cell culture" and "culturing of cells" refer to the maintenance and propagation of cells and preferably human, human-derived and animal cells in vitro.

"Cell culture medium" is used for the maintenance of cells in culture in vitro. For some cell types, the medium may also be sufficient to support the proliferation of the cells in culture. A medium according to the present invention provides nutrients such as energy sources, amino acids and anorganic ions. Additionally, it may contain a dye like phenol red, sodium pyruvate, several vitamins, free fatty acids, antibiotics, anti-oxidants and trace elements.

For culturing the stem cells according to the present invention any standard medium such as Iscove's Modified Dulbecco's Media (IMDM), alpha-MEM, Dulbecco's Modified Eagle Media (DMEM), RPMI Media and McCoy's Medium is suitable before reprogramming. Ones the cells have been reprogrammed, they can in a preferred embodiment be cultured in embryonic stem cell medium.

Preferably, the medium additionally comprises one or more additives selected from the group consisting of vitamin D3 (1,25-dihydroxyvitamin D3, cacitriol), resveratrol (trans-3, 4', 5-trihydroxystilbene), reversine (2-(4-morpholinoanilino)- N6-cyclohexyladenine), vitamin E (RRR-α-tocopherol), valproic acid (dekapene, valproate, valrelease), EGCG (epigallocatechin-3-gallat) and selenium. Preferably, two of the afore-mentioned additives are present, more preferably three of the afore-mentioned additives are present, even more preferably four of the afore-mentioned additives are present, particularly preferably five of the afore-mentioned additives are present and most preferably all of the aforementioned additives are present.

These media are well-known to a person skilled in the art and may be purchased from companies such as Cambrex, Invitrogen, Sigma-Aldrich or Stem Cell Technologies.

The medium may in a particular embodiment further comprise a serum component such as horse serum, human serum or fetal calf serum (FCS). Preferably, the medium contains FCS. If present, the serum is present in a concentration of 1 -20%, preferably of 3-18%, more preferably of 5-15%, even more preferably of 8-12% and most preferably of 10%. Alternatively the serum component may be replaced by any of several standard serum replacement mixtures which typically include insulin, albumin and lecithin or cholesterol.

A "culture vessel" is any vessel which is suitable for growing cells in a culture medium, in particular selected from, but not limited to, agar, matrigel and collagen, either in fluid phase or adhered to an interior surface of the container. Types of such specialized vessels include roller bottles, spinner flasks, petri dishes and tissue flasks. Culture vessels are designed to be incubated in temperature, humidity and gas controlled environments to facilitate maximum cell or tissue growth. Generally, a layer of cell culture medium or agar covers the growing surface. The portion of the vessel not utilized as a growing surface encloses the interior gaseous environment which surrounds the cell culture. Cells, tissues, microorganisms and the like typically are introduced into the interior of cell culture vessels through an opening in the vessel. After introduction of the cells the opening may be closed such that the cells are not in contact with the environment during the culturing of the cells.

In a preferred embodiment of the present invention, the culture vessel is treated for tissue culture, which means that the surface of the culture vessel is treated such that cells which usually grow in an adherent state grow adherently, but cells which grow in suspension do not adhere or adhere only loosely. Such treatment may involve the irradiation of the vessel or the coating of the vessel, for example with a special plastic, polymer or nanostructure or with proteins of the extracellular matrix. Such vessels can comprise tissue culture dishes and tissue culture flasks and are available from different suppliers such as Becton Dickinson, Greiner, Sigma and TPP.

The present invention also relates to a process for the production of cells exhibiting a multipotent, preferably a multipotent, stem cell character, wherein a process according to the above is carried out.

The present invention also relates to a process for improving the expansion of stem cells in vitro, wherein a process according to the present invention is carried out. Thus, the present invention provides the advantage of prolonging expansion of stem cells, since the present invention keeps the cells to be expanded in a dedifferentiated status.

The present invention also relates to a process for the rejuvenation of aged cells, wherein a process according to the present invention is carried out.

The present invention also relates to a process for inducing the dedifferentiation of cells, wherein a process according to the present invention is carried out and wherein in particular the cells to be reprogrammed are differentiated cells, in particular lineage-committed cells.

The present invention also relates to a process for reprogramming recipient cells of a mammal in vivo comprising introducing reprogramming mRNA molecules into the recipient cells of the mammal wherein the reprogramming mRNA molecules are encoding at least one reprogramming protein selected from the group consisting of Ronin, Oct4, Klf4, Sox2, Nanog and TERT. Thus, the present invention also relates to an in vivo method for reprogramming recipient cells of a mammal which employ the reprogramming mRNA molecules as identified above for the in vitro embodiment of the present invention. The present invention relating to the process for reprogramming recipient cells of a mammal in vivo thereby foresees to introduce the reprogramming mRNA molecules directly into at least one cell of a recipient mammal that means into at least one recipient cell by suitable means such as gene gun insertion. Such a process allows the production of a mammal in particular a human being which has at least one reprogrammed cell. Thus, this embodiment of the present invention provides terrifically improved therapeutic application, in particular in the regenerative medicine and/or in replacement therapy. Thus, the present invention relates to a method of treating a mammal, in particular human being wherein reprogramming mRNA molecules are introduced into at least one cell of the recipient mammal and wherein the reprogrammed mRNA molecules are encoded at least by encoding at least one reprogramming protein selected from the group consisting of Ronin, Oct4, Klf4, Sox2, Nanog and TERT.

The present invention also relates to a multipotent, in particular a pluripotent, stem cell prepared according to any one of the methods of the present invention. Thus, the reprogrammed cells according to the present invention are preferably multipotent, in particular pluripotent stem cells. In a preferred embodiment the cells obtained according to the present invention are distinguished from the cells used as a starting material in step a) of the present invention by lack of one or more markers. These cells may in a preferred embodiment be also characterized by a particular methylation pattern, by the expression of oct4, sox2, nanog, hTERT or klf4. expression of oct4, which is not found in somatic cells like fibroblasts, becomes expressed after transfecting mRNA (klf4, sox2 and oct4) in human fibroblasts, by improved growth potential; and by increased differentiation potential. For pluripotency the formation of embryoid bodies, production of cells of all three germ layers in vitro and in vivo. Examples thereof are illustrated in the accompanying figures and example.

The reprogrammed cells obtained by the present processs may preferably be characterized by the expression or non-expression of certain surface markers. These surface markers are usually given a cluster of differentiation (CD) designation which describes groups of immunophenotypical surface characteristics of cells. Usually CD molecules are membrane-bound glycoproteins which are recognized by a cluster of monoclonal antibodies that display the same cellular reactivity. These surface molecules may for example be detected by means of a flow cytometer such as a fluorescenceactivated cell sorter (FACS) manufactured by companies such as Becton Dickinson. Methods to identify cells according to the present invention are methods to detect cell-specific proteins, methods to detect cell-specific transcription factors or methods to detect physiological or morphological changes. All of these methods are per se well-known in the prior art. In particular, these methods use well-known protein or DNA or RNA detection processes and/or the reporter gene assays. Of course, immunoassays or assays using fluorescently or radioactively labelled proteins or nucleic acids including methods to measure enzymatic activities can be employed. Methods to detect morphological changes may include staining methods, visually based methods or the like.

Reprogrammed cells according to the present invention may be used for therapeutic, diagnostic or scientific purposes. In particular, these cells may be used in regenerative medicine and/or for replacement therapy.

The term "cell" not only refers to a single cell but also encompasses a cell line, a cell population or a cell clone.

The term "stem cells" refers to cells which have retained the capacity to proliferate and differentiate into one or different cell types. Stem cells created in accordance with the present invention are preferably pluripotent stem cells, i.e. stem cells which have retained the capacity to differentiate into distinct cell lineages and cell types or multipotent stem cells, retaining a more restricted differentiation potential.

It is understood that the term "stem cells" in accordance with the invention does not comprise human embryos. Furthermore, it is understood that the term "stem cells" does not comprise pluripotent stem cells which have been directly derived from a human embryo. Embryonic stem cells which have been derived from publicly available and previously established stem cell lines are understood to fall within the meaning of the term "stem cells" as used by the present invention.

A "stem cell", as used herein, refers to any self-renewing pluripotent cell or multipotent cell or progenitor cell or precursor cell that is capable of differentiating into one or multiple cell types. Stem cells are thus cells able to differentiate into one or more than one cell type and have preferably an unlimited growth potential. Stem cells include those that are capable of differentiating into cells of osteoblast lineage, a mesenchymal cell lineage (e. g. bone, cartilage, adipose, muscle, stroma, including hematopoietic supportive stroma, and tendon). "Differentiate" or "differentiation", as used herein, refers to the process by which precursor or progenitor cells (i. e., stem cells) differentiate into specific cell types, e. g., osteoblasts. Differentiated cells can be identified by their patterns of gene expression and cell surface protein expression. "Dedifferentiate" or "dedifferentiation", as used herein, refers to the process by which lineage-committed cells (e. g., myoblasts or osteoblasts) reverse their lineage commitment and become precursor or progenitor cells (i. e., multipotent or pluripotent stem cells). Dedifferentiated cells can for instance be identified by loss of patterns of gene expression and cell surface protein expression associated with the lineage committed cells.

A "lineage-committed cell" as used herein, refers to any cell that has or will differentiate into a particular cell type or related cell types. Lineage committed cells include, for example, osteoblasts, myoblasts, chrondrocytes, and adipocytes.

Totipotent stem cells are able to create all cell types of the body, including placental cells. The earlier stage foetal stem cells are considered totipotent, as well as the fertilised egg. They also have the ability to replicate in unlimited numbers without losing their potential.

Pluripotent stem cells are able to create cells of all three germ layer, namely the ecto-, endo- and mesoderm. They also have the ability to replicate in unlimited numbers without losing their potential.

Multipotent stem cells are able to produce cells of one or more germ layers or several types of tissues. They often have an already limited self-renewal ability.

Stem cells are thus cells able to differentiate into one or more than one cell type and have preferably an unlimited growth potential.

Progenitor cells can differentiate into one or more cell types but have a limited growth potential.

Adult stem cells are stem cells derived from an adult organism and might be multipotent or pluripotent.

Embryonic stem cells are derived from the inner mass of a blastula and are pluripotent. Embryonic stem cells are unique because they can develop into nearly all cell types, an attribute called pluripotency. But to access these cells, researchers must destroy a viable embryo. In this patent we are describing a way to create cells, with the characteristics of embryonic stem cells, without the need to destroy embryos or the use of embryonic stem cells.

Induced pluripotent stem cells "IPS" cells are pluripotent stem cells artificially derived from non-pluripotent cells, including somatic cells, adult multipotent stem cells or progenitor cells. In principal every cell containing a nuclei can be used as source for IPS cells.

Further preferred embodiments of the present invention are the subject matter of the subclaims.

The sequence listing show the prior art DNA sequences encoding the mRNA nucleotide sequence molecules used in the present invention:

**Table 1**

| **SEQ ID No.** | **gene** | **accession-number** | **length** |
|---|---|---|---|
| **1** | **hNanog** | **NM_024865** | **2098** |
| **2** | **mNanog** | **NM_028016** | **1356** |
| **3** | **rNanog** | **NM_001100781** | **2358** |
| **4** | **hSox2** | **NM_003106** | **2518** |
| **5** | **mSox2** | **NM_011443** | **2457** |
| **6** | **rSox2** | **NM_001109181** | **2323** |
| **7** | **hOct4** | **NM_002701** | **1411** |
| **8** | **mOct4** | **NM_013633** | **1346** |
| **9** | **hKlf4** | **NM_004235** | **2949** |
| **10** | **mKlf4** | **NM_010637** | **3057** |
| **11** | **rKlf4** | **NM_053713** | **2393** |
| **12** | **hTERT** | **NM_198253** | **4018** |
| **13** | **mTert** | **ENSMUSG00000021611** | **4237** |
| **14** | **rTERT** | **NM_053423** | **3378** |
| **15** | **hRonin** | **NM_020457** | **1903** |
| **16** | **mRonin** | **NM_021513** | **1832** |
| **abbreviations: h = human; m = mouse; r = rat** | | | |

The designations marked "NM" and ENSMUSG refer to the NM(NCBI)- and ENSMUSG-accession numbers as given under the publically available website http://www.ncib.nlm.nih.gov and http://www.ensembl.org.

SEQ ID No. 17 and 18 show the DNA sequence of primers used for cloning the human Nanog gene.

SEQ ID No. 19 and 20 show the DNA sequence of primers used for cloning the human Klf4 gene.

SEQ ID No. 21 and 22 show the DNA sequence of primers used for cloning the human Sox2 gene.

SEQ ID No. 23 and 24 show the DNA sequence of primers used for cloning the human Oct4 gene.

SEQ ID No. 25 and 26 show the DNA sequence of primers used for cloning the human Tert gene.

SEQ ID No. 27 and 28 show the DNA sequence of primers used for cloning the GFP gene.

The present invention will now be illustrated in more detail by way of an example and figures.

The figures show:

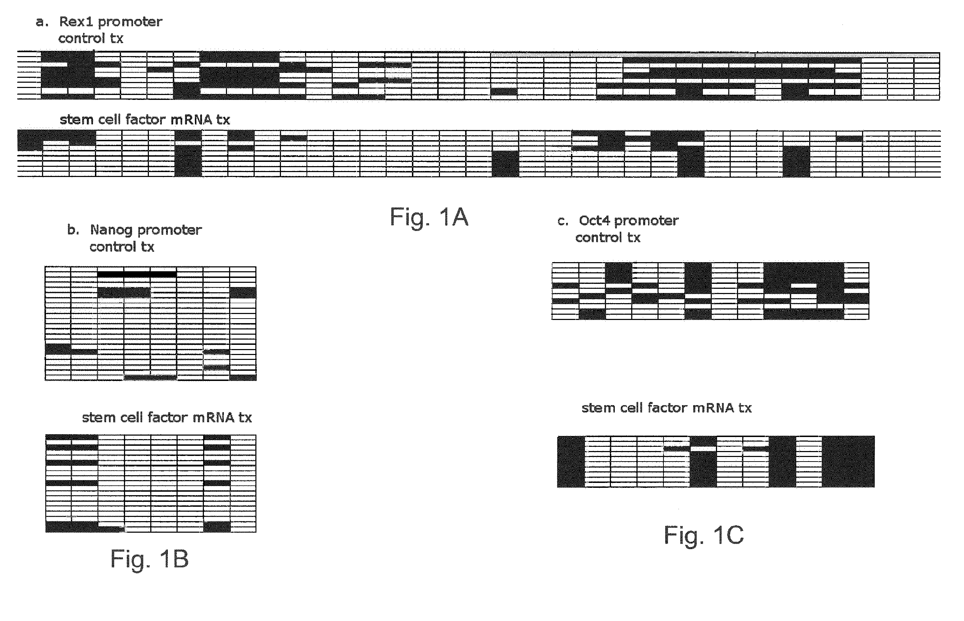

Figure 1: Promotor methylation of rex1, nanog and oct4 in human fibroblasts before and after reprogramming (Figure 1A: Rex; Figure 1AB: Nanog; Figure 1C: Oct4). CpG island are represented as squares. Black squares represent methylated and white squares represent unmethylated CpG islands. All samples are primary fibroblasts from a young male donor.

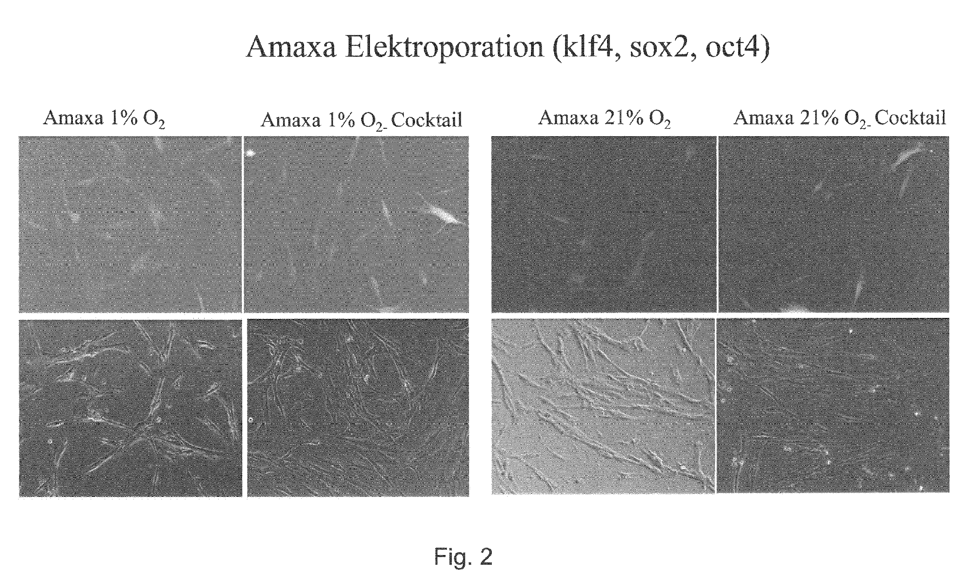

Figure 2: Fluorescence and morphology after amaxa elektroporation (klf4, sox2, oct4): Human primary fibroblasts were transfected with 0,6 µg/µl mRNA per factor and 0,2 µg/µl GFP mRNA to visualize the transfection effciency. Top line are fluorescence pictures and the bottom line shows the morphology of the cells after 2 days.

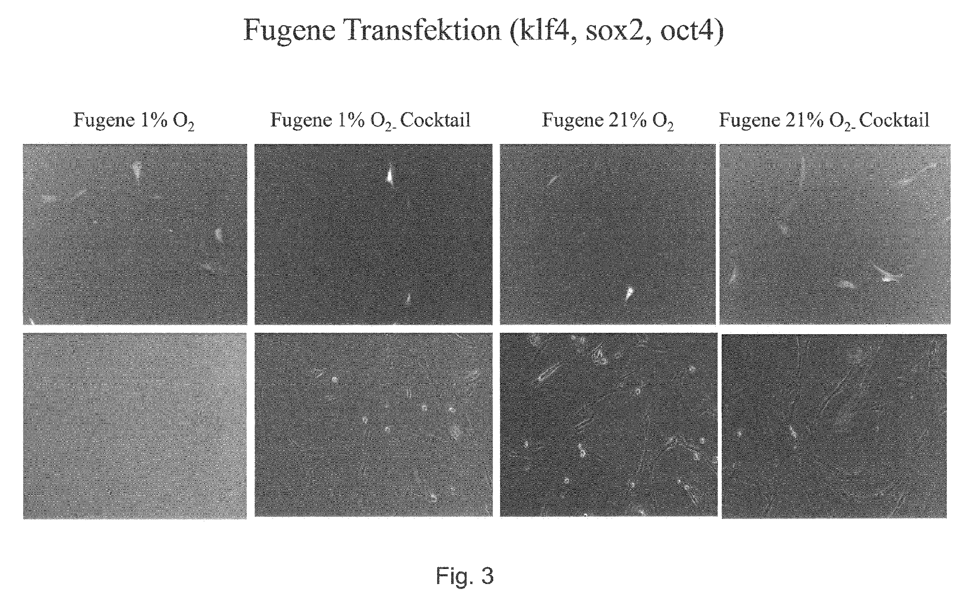

Figure 3: Fluorescence and morphology after Fugene transfection (klf4, sox2, oct4): Human primary fibroblasts were transfected with 0,6 µg/µl mRNA per factor and 0,2 µg/µl GFP mRNA to visualize the transfection effciency. Top line are fluorescence pictures and the bottom line shows the morphology of the cells after 7 days.

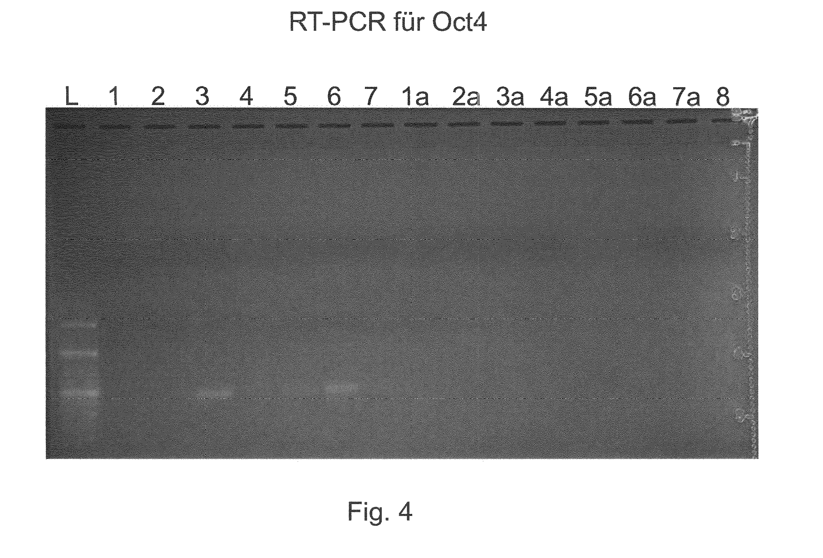

Figure 4: RT-PCR for Oct4: L: 20 bp Ladder (Fermentas); 1 hOct4 hypoxy factors1x transfected; 2: hOct4 hypoxy factors 6x transfected; 3: hOct4 hypoxy factors 6x transfected +Cock; 4: hOct4 normoxy factors 1x transfected; 5: hOct4 normoxy factors 6x transfected; 6: hOct4 normoxy factors 6x transfected +Cock; 7: hOct4 normoxy factors 6x transfected +Cock+MG-132; 1 a: hOct4 RNA after DNase digestion; 2a: hOct4 RNA after DNase digestion; 3 a: hOct4 RNA after DNase digestion; 4a: hOct4 RNA after DNase digestion; 5a: hOct4 RNA after DNase digestion; 6a: hOct4 RNA after DNase digestion; 7a: hOct4 RNA after DNase digestion; 8: Oct4 negative control.

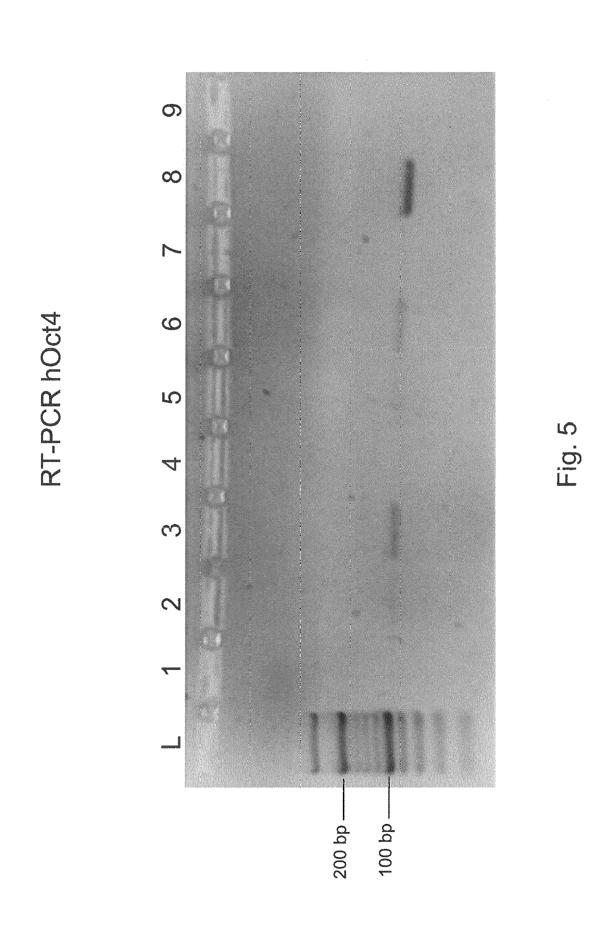

Figure 5: RT-PCR for hOct4: L: 20 bp Ladder (Fermentas); 1 : hOct4 hypoxy factorslx transfected (human fibroblasts); 2: hOct4 hypoxy factors 6x transfected (human fibroblasts mit mRNA transfected); 3: hOct4 hypoxy factors 6x transfected +Cocktail (human fibroblasts transfected with mRNA and chemical reprogramming cocktail); 4: hOct normoxy factors 1x transfected; 5: hOct4 normoxy factors 6x transfected; 6: hOct4 normoxy factors 6x transfected + cocktail, 7: hOct4 normoxy 6x transfected factors + cocktail + MG-132; 8: hOct4 positive control (plasmid with hKlf4), 9: hOct4 negative control (water).

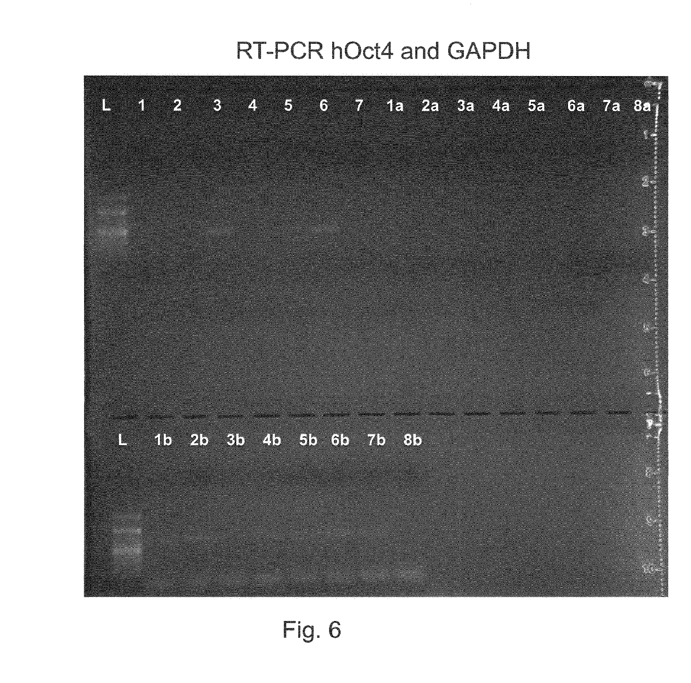

Figure 6: RT-PCR for hOct4 and GAPDH: 1 hOct4 hypoxy factorslx transfected; 2 hOct4 hypoxy factors 6x transfected; 3 hOct4 hypoxy factors 6x transfected +chemical cocktail; 4 hOct4 normoxy factors 1x transfected; 5 hOct4 normoxy factors 6x transfected; 6 hOct4 normoxy factors 6x transfected + chemical cocktail; 7 hOct4 normoxy factors 6x transfected + chemical cocktail + MG-132; 1 a hOct4 RNA after DNase digestion; 2a hOct4 RNA after DNase digestion; 3a hOct4 RNA after DNase digestion; 4a hOct4 RNA after DNase digestion; 5a hOct4 RNA after DNase digestion; 6a hOct4 RNA after DNase digestion; 7a hOct4 RNA after DNase digestion; 8a Oct4 negative control; 1 b GAPDH hypoxy factors 1x transfected; 2b GAPDH hypoxy factors 6x transfected; 3b GAPDH hypoxy factors 6x transfected + chemical cocktail; 4b GAPDH normoxy factors 1x transfected; 5b GAPDHnormoxy factors 6x transfected; 6b GAPDH normoxy factors 6x transfected + chemical cocktail; 7b GAPDH normoxy factors 6x transfected + chemical cocktail + MG-132; 8b GAPDH negative control.

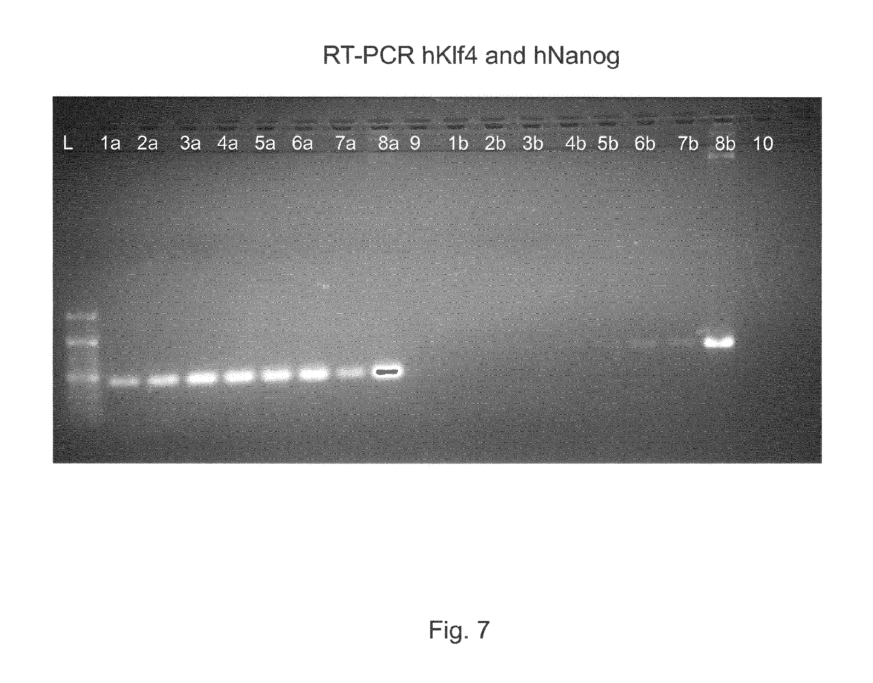

Figure 7: RT-PCR for hKlf4 and hNanog: L: 20 bp Ladder (Fermentas); 1a: hKlf4 hypoxy factors1x transfected (human fibroblasts); 2a: hKlf4 hypoxy factors 6x transfected (human fibroblasts with transfected mRNA); 3 a: hKlf4 hypoxy factors 6x transfected + cocktail (human fibroblasts transfected with mRNA + chemical reprogramming cocktail); 4a: hKlf4 normoxy factors 1x transfected; 5a: hKlf4 normoxy factors 6x transfected; 6a: hKlf4 normoxy factors 6x transfected + cocktail, 7a: hKlf4 normoxy 6x transfected factors + cocktail + MG-132; 8a: hKlf4 pos. control (plasmid with hKlf4), 9a: hKlf4 neg. control (water) 1b: hNanog hypoxy factorslx transfected (human fibroblasts); 2b: hNanog hypoxy factors 6x transfected (humane fibroblasts mit mRNA transfected); 3b: hNanog hypoxy factors 6x transfected + cocktail (human fibroblasts transfected with mRNA + chemical reprogramming cocktail); 4b: hNanog normoxy factors 1x transfected; 5b: hNanog normoxy factors 6x transfected; 6b: hNanog normoxy factors 6x transfected + cocktail, 7b: hNanog normoxy 6x transfected factors + cocktail + MG-132; 8b hNanog pos. control (plasmid with hNanog), 10 hNanog neg, control (water).

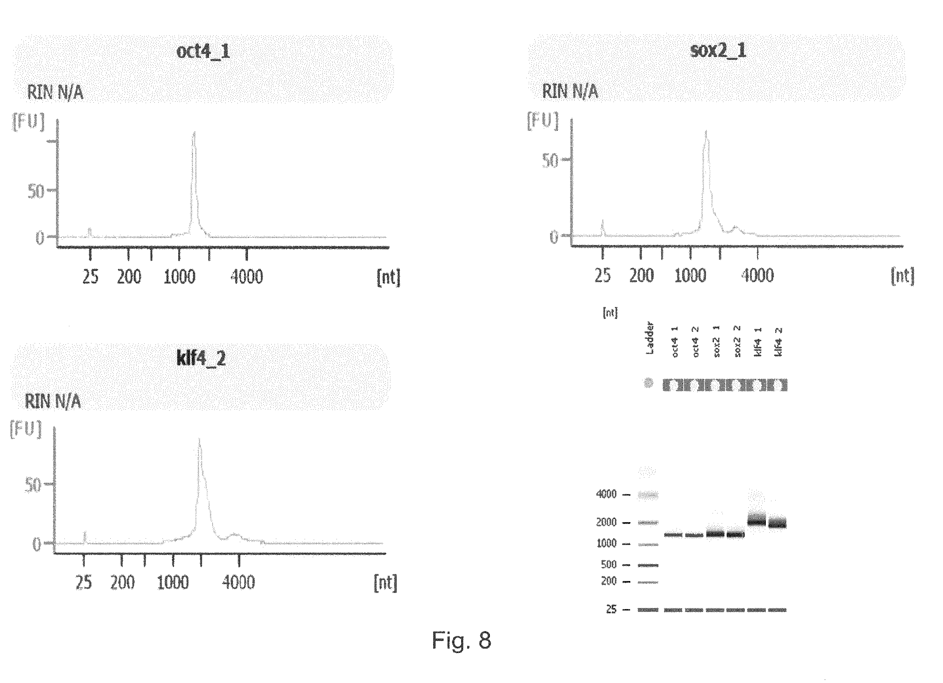

Figure 8: Results from RNA BioAnalyzer Analysis (Agilent Nano Chip; Agilent Technologies) for oct4, sox2, and klf4:

Further preferred embodiments of the invention are reflected by the below consecutively numbered sentences:
1. A process for preparing reprogrammed cells in vitro, comprising
   a) providing cells to be reprogrammed,
   b) introducing reprogramming mRNA molecules into the cells provided in step a), wherein the reprogramming mRNA molecules are encoding at least one reprogramming protein selected from the group consisting of Ronin, Oct4, Klf4, Sox2, Nanog and TERT,
   c) culturing the cells obtained in step b) in a cell culture medium and under a condition suitable to allow translation of the transfected reprogramming mRNA molecules so as to obtain reprogrammed cells.
2. The process according to embodiment 1, wherein the condition suitable to allow translation is an oxygen content in the cell culture medium from 0.5 % to 21 %.
3. The process according to embodiments 1 or 2, wherein the condition suitable to allow translation is a temperature from 30°C to 38°C.
4. The process according to any one of the preceding embodiments, wherein the cell culture medium has a glucose content of 0.1 g/l to 4.6 g/l.
5. The process according to any one of the preceding embodiments, wherein the cell culture medium contains at least one inducing substance selected from the group consisting of reversin, resveratol, selenium, selenium containing compounds, EGCG ((-)-epigallocatechin-3-gallate), valporic acid, salts of valporic acid and sodium valproate.
6. The process according to any one of embodiments 1 to 5, wherein the cell culture medium contains at least one transient proteolysis inhibitor.
7. The process according to embodiment 6, wherein the transient proteolysis inhibitor is selected from the group consisting of protease inhibitor, proteasome inhibitor and lysosome inhibitor.
8. The process according to any one of the preceding embodiments, wherein the cells to be reprogrammed are human cells.
9. The process according to any one of the preceding embodiments, wherein the cells to be reprogrammed are non-embryonic stem cells.
10. A process for the production of cells exhibiting a pluripotent or multipotent stem cell character, wherein a process according to any one of embodiments 1 to 9 is carried out.
11. A process for inducing the dedifferentiation of cells, wherein a process according to any one of embodiments 1 to 9 is carried out.
12. A process for improving the expansion of non-embryonic stem cells in vitro, wherein a process according to any one of embodiments 1 to 9 is carried out.
13. A process for the rejuvenation of aged cells, wherein a process according to any one of embodiments 1 to 9 is carried out.
14. A multipotent or pluripotent stem cell prepared according to any one of the methods of embodiments 1 to 10.
15. A process for reprogramming recipient cells of a mammal in vivo comprising introducing reprogramming mRNA molecules into the recipient cells of the mammal wherein the reprogramming mRNA molecules are encoding at least one reprogramming protein selected form the group consisting of Ronin, Oct4, Klf4, Sox2, Nanog and TERT.

### Example

### 1. Preparation of reprogramming mRNA

### 1. A) Preparation of mRNA expression plasmids

For the construction of plasmids comprising the target genes in a vector with the T7-promoter for in *vitro* transcription, using PCR, digest sites before the start-codon and after the stop codon were designed for direct cloning of the gene inserts (see Table 1) in the right direction into the pCR@II-Vector (Invitrogen).

The primers used are listed in Table 2.

**Table 2**

| Name gen | New digest sites for restriction enzymes | Primer sequence 5'-3' | Product size |
|---|---|---|---|
| Nanog_human | XbaI & Notl | SEQ ID No. 17 | 918 bp |
| Nanog_human | SpeI & Clal | SEQ ID No. 18 | |
| Klf4_human | XbaI & Notl | SEQ ID No. 19 | 637 bp |
| Klf4_human | HindIII & Clal | SEQ ID No. 20 | |
| Sox2_human | XbaI & BamHI | SEQ ID No. 21 | 996 bp |
| Sox2_human | HindIII & Clal | SEQ ID No. 22 | |
| Oct4_human | XbaI & Notl | SEQ ID No. 23 | 1100 bp |
| Oct4_human | BamHI & Clal | SEQ ID No. 24 | |
| Tert_human | XbaI & Notl | SEQ ID No. 25 | 783 bp |
| Tert_human | HindIII & Clal | SEQ ID No. 26 | |
| GFP | XbaI & Notl | SEQ ID No. 27 | 724 bp |
| GFP | HindIII & Clal | SEQ ID No. 28 | |

A PCR for obtaining the desired digest sites was carried out with the Platinum Taq-Polymerase (Invitrogen).

### Preparation of PCR-reaction on ice:

| | |
|---|---|
| 5 µl | 10x PCR Buffer |
| 1 µl | 10 mM dNTP mixture 0.2 mM each |
| 1.5 µl | 50 mM MgCl2 |
| 1 µl | Primer mix (10 µM each) 0.2 µM each |
| 1 µl | Template DNA |
| 0.2 µl | Platinum^{®} Taq DNA Polymerase 1.0 unit |
| to 50 µl | DEPC-H₂O |

### PCR-Program (Biometra Tprofessional):

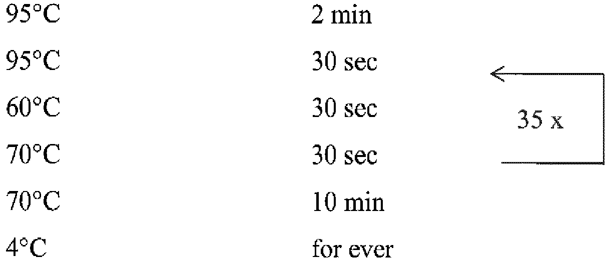

After electrophoresis in a 1 % TAE-agarose-gel, specific DNA-bands (for their size see Table 2) were cut out and the DNA extracted and purified with the QIAquick Gel Extraction Kit (from Qiagen)

### Procedure:

- excise the DNA fragment from the agarose gel with a scalpel
- weigh the gel slice
- add 3 volumes of Buffer QG to 1 volume of gel (For example, add 300 µl of Buffer QG to each 100 mg of gel)
- incubate at 50°C for 10 min (or until the gel slice has completely dissolved)
- add 1 gel volume of isopropanol to the sample and mix
- place a QIAquick spin column in a provided 2 ml collection tube
- to bind DNA, apply the sample to the QIAquick column, and centrifuge for 1 min at 13,000 rpm
- discard flow-through
- add 0.5 ml of Buffer QG to QIAquick column and centrifuge for 1 min at 13,000 rpm
- to wash, add 0.75 ml of Buffer PE to QIAquick column and centrifuge for 1 min at 13,000 rpm
- discard the flow-through
- centrifuge the QIAquick column for an additional 1 min at 13,000 rpm
- place QIAquick column into a 1.5 ml micro centrifuge tube
- to elute DNA, add 50 µl of H20 to the centre of the QIAquick membrane, let the column stand for 1 min, and then centrifuge for 1 min at 13,000 rpm

Digest of pCRII-Vector with the specific restriction enzymes for cloning of target-DNA

### Strategy for digest of vector pCRII:

vector pCRII digest with XbaI and SpeI for target gene human Nanog
vector pCRII digest with XbaI and HindIII for target gene human Klf4
vector pCRII digest with Xbal and BamHI for target gene human Sox2
vector pCRII digest with XbaI and BamHI for target gene human Oct4
vector pCRII digest with XbaI and HindIII for target gene human TERT
vector pCRII digest with XbaI and HindIII for target gene GFP

### Preparation of digest on ice:

- 5 µl 10x Buffer
- ≥2 µg plasmid
- 2 µl restriction enzyme
- to 50 µl DEPC-water

- incubation for 1 h at 37°C (thermo block; Eppendorf)
- heat inactivation for 20 min by the specific temperature of the enzyme

Ligation of the target-gene inserts DNA with the linear vector DNA with the T4-DNA-Ligase (Fermentas)

### Preparation of the ligation on ice:

- 50-400 ng vector DNA
- 50-400 ng insert DNA
- 2 µl 10x Buffer
- 0.2 µl (1U) T4-DNA Ligase
- to 20 µl DEPC-water

- vortex the tube and spin down in a micro centrifuge for 3-5 seconds
- incubate on room temperature for 1 h
- heat inactivation for 20 min

### 1. B) Transformation of the plasmids with chemical competent DH5α-bacteria

- add 1 µl of the plasmids to 10 µl chemical competent DH5α-bacteria
- incubation for 30 min on ice
- heat shock at 42°C for 45 sec
- add 250 µl SOC-Medium to the bacteria
- incubation at 37°C for 1h with shaking
- plate out the bacteria solution on LB-kanamycin-agarose-plate and incubate the plate for 12 hours in 37°C incubator
- pick one colony of the bacteria and put the colony in 5 ml LB-medium and incubate the solution for 12 hours in a bacteria incubator with shaking

### 1. C) Plasmid DNA purification with the NucleoSpin® Plasmid -KIT (Machery&Nagel)

- 5 ml of a saturated E. coli LB culture, pellet cells in a standard bench top micro centrifuge for 30 sec at 11,000 x g.
- discard the supernatant
- for cell lysis add 250 µl Buffer A1. Re-suspend the cell pellet completely by pipetting up and down
- add 250 µl Buffer A2
- mix gently by inverting the tube 10 times and Incubate at room temperature for up to 5 min
- add 300 µl Buffer A3. Mix thoroughly by inverting the tube 10 times
- centrifuge for 5 min at 11,000 x g at room temperature
- place a NucleoSpin® Plasmid Column in a collection tube (2 ml) and load a 750 µl of the supernatant onto the column.
- centrifuge for 1 min at 11,000 x g.
- discard flow-through and place the NucleoSpin® Plasmid Column back into the collection tube (2 ml)
- for wash the membrane with DNA: Add 600 µl Buffer A4 (supplemented with ethanol).
- centrifuge for 1 min at 11,000 x g.
- discard flow-through and place the NucleoSpin® Plasmid Column back into the collection tube (2 ml)
- dry silica membrane: centrifuge for 2 min at 1 1,000 x g and discard the collection tube (2 ml)
- elute DNA: place the NucleoSpin® Plasmid Column in a 1.5 ml micro centrifuge tube and add 50 µl H20 and incubate for 1 min at room temperature.
- centrifuge for 1 min at 11,000 x g
- measure the DNA-concentration with the nanodrop
- DNA store at -20°C

### 1. D) Linearisation of the pCRII-hoct4, pCRII-hSox2, pCRII-hKlf4 plasmids for the in vitro transcription

- pCRII-hoct4 digest with BamHI
- pCRII-hSox2 digest with HindIII
- pCRII-hKlf4 digest with HindIII

### Preparation of digest on ice:

- 5 µl 10x Buffer
- 2 µg Plasmid
- 2 µl Restriction-Enzyme
- to 50 µl DEPC-H₂0

- incubation for 1 h at 37°C (thermo block; Eppendorf)
- heat inactivation for 20 min by the specific temperature of the enzyme
- heat inactivate both enzyme for 20 min at 80°C

### 1. E) Purification of the linearized pCRII-hoct4, pCRII-hSox2, pCRII-hKlf4 plasmids with QlAquick PCR Purification Kit (from Qiagen)

- add 5 volumes of Buffer PB to 1 volume of the digest sample and mix
- place a QIAquick spin column in a provided 2 ml collection tube
- to bind DNA, apply the sample to the QIAquick column and centrifuge for 60 s
- discard flow-through
- place the QIAquick column back into the same tube
- to wash, add 0.75 ml Buffer PE to the QIAquick column and centrifuge for 60 s
- discard flow-through and place the QIAquick column back in the same tube
- to dry the membrane, centrifuge the column for an additional 1 min
- place QIAquick column in a 1.5 ml micro centrifuge tube
- to elute DNA, add 10 µl H20 to the centre of the QIAquick membrane, let the column stand for 1 min, and centrifuge the column for 1 min
- measure the DNA-concentration with the nanodrop
- store DNA at -20°C

### 1. F) in vitro transcription of the linearized pCRII-hoct4, pCRII-hSox2, pCRII-hKlf4 plasmids with mMessage mMachine high yield capped RNA Transcription Kit (from Ambion) and add a poly(A) tail to RNA transcripts (Poly(A)-Tailing Kit from Ambion)

### Preparation of the in vitro transcription (mMESSAGE mMACHINE reaction):

- to 20 µl nuclease-free water
- 10 µl 2x NTP/CAP
- 2 µl 10x reaction buffer
- 1 µg linear template plasmid
- 2 µl enzyme mix

- pipette the mixture up and down gently
- short centrifugation to collect the reaction mixture at the bottom of the tube
- incubation for 2 h at 37°C
- add 1 µl TURBO DNase to the mRNA and mix well
- incubate at 37°C for 15 min
- direct after the DNase-treated reaction add the poly(A)-tail to the mRNA

### Preparation for poly(A)-tail reaction:

- 20 µl mMESSAGE mMACHlNE reaction
- 36 µl nuclease-free water
- 20 µl 5x E-PAP Buffer
- 10 µl 25 mM MnCl2
- 10 µl mM ATP
- 4 µl E-PAP

- mix gently
- incubate at 37°C for 1 h
- place reaction on ice
- lithium chloride precipitation of the mRNA with poly(A)-tail:
- add 30 µl LiCl Preciptitation Solution
- mix thoroughly
- chill for 30 min at -20°C
- centrifuge at 4°C for 15 min at maximum speed to pellet the RNA
- remove the supernatant
- wash the pellet once with 1 ml 70% ethanol
- centrifuge at 4°C for 15 min at maximum speed to pellet the RNA
- remove the 70% ethanol
- air-dry the pellet
- solve the RNA-Pellet with 30-50 ml nuclease-free-water
- the concentration of the RNA is measured with the nanodrop
- the quality of the RNA is measured with the Agilent nano-chip

### 2. Preparation of target cells

### 2. A) Preparation of mouse and rat MSCs

- kill the rat or mouse with CO2-gassing
- spray the rats and mice with 70% Ethanol to kill of bacteria and fungal spores
- remove the coat from the skin
- remove the back legs cleanly at the hip joint
- under sterile conditions, remove all soft tissue and separate the bones
- remove the growth plates both femur and tibia
- insert holes with a needle into the bones on both ends
- place the bones in eppendorf tubes (for mice) or in falkon tubes (for rats) and spin at 2000 rpm for 1 min (all of the bone marrow will be deposited in the bottom of the tube sitting in spacers); remove the bones and spacer
- re-suspend the marrow in 0,5 ml medium (DMEM low glucose; 10 % FCS; 1 % Pen/Strep)
- disseminate the marrow of one leg from a rat in 1 T-75 flask with 12 ml medium
- disseminate the marrow of two legs from a mouse in 1 10 cm petri-dish with 12 ml medium

### 2. B) Preparation of human MSC

- get a bone marrow aspirate
- re-suspend in DMEM (low glucose, 10%FCS)
- plate out in a tissue culture flask
- growth for 5 days and then change media
- thereafter change the medium twice weekly
- culture in tissue culture flask until 80% confluency and subcul ture until needed

### 2. C) Preparation of mouse and rat fibroblasts

- cut of the tail
- shave the tail with a scalpel
- spray the tail with 70% ethanol to kill of bacteria and fungal spores
- remove the skin from the tail
- place the skin in a 10 cm petri dish
- under sterile conditions, cut the skin in small pieces (5 x 5 mm)
- digest the skin-pieces with 0,05 % Trypsin EDTA for 20 min in 37°C CO2-Incubator
- wash the trypsin from the skin-dishes with medium (DMEM high glucose, 10 % FCS, 1 % Pen/Strep), remove the medium and wash again for 3-4 times
- then incubate the skin pieces in 37°C CO2-Incubator and change the medium every day, after 1 week the fibroblasts growth out

### 2. D) Preparation of human fibroblasts

- place prepuce in petri dish
- wash two times with PBS
- remove fat
- wash with PBS one time
- cut the skin in small pieces (2 mm)
- add 10ml) dispase (2 U/ml)
- incubate at 4°C for 16-18 h
- discard dispase solution
- add 5 ml PBS
- seperate dermis and epidermis
- transfer dermis to new dish and add 10ml collagenease (500 U/ml)
- make very small pieces
- transfer pieces into a 50 ml falcon tube with solution and incubate for 45 min (37°C; 5% CO2)
- centrifuge at 1000rpm for 5 min
- discard supernatant and resuspend pellet in DMEM (10% FCS)
- centrifuge again
- reuspend pellet in 2 ml DMEM (10% FCS) and transfer into a T75 cell culture flacon
- expanded cells until use

### 3. mRNA Transfection

### 3. A) mRNA-transfection of human fibroblasts, human MSC, rat fibroblasts, rat MSC, mouse fibroblasts and mouse MSC with AMAXA, FugeneHD, Lipofectamine™ LTX Reagent and PLUS™ Reagent

### 1) AMAXA-Transfection with the Human Dermal Fibroblast Nucleofector® Kit (6 well-plate):

### Preparation for one electroporation in certified Amaxa cuvette:

- mRNA of oct4, sox2 and klf4 in sum of 2 µg
- 4x105 cells
- 100 µl Nucleofector® Solution
- choose the program U-023 by the Amaxa-electroporation-machine
- re-suspend the cells in the cuvette with 500 µl fibroblast or MSC-Medium
- provide 5 ml fibroblast or MSC-Medium with or without Cocktail in every well of a 6 well-plate
- plate out 100.000 cells in each well of a 6 well-plate

### Cocktail composition:

- Resveratrol 0,05 mM
- Selenium 0,1 µM
- EGCG ((-)-Epigallocatechin gallate) 0,01 µM
- TSA (Trichostatin A) 0,015 µM
- Reversin 1 µM
- VPA (2-Propylpentanoic acid free acid) 6 µM
- 5'Aza (5-Aza-2'-deoxycytidine) 1 µM

- at day 1: the cocktail is with 5'Aza and without TSA; after 48 hours 5'Aza replaced by TSA
- every 72 hours new transfection with FuGENE® HD Transfection Reagent (Roche)

### 2) Transfection FuGENE® HD Transfection Reagent (Roche)

>

### - before transfection: change to antibiotic free medium

### Transfection procedure

- Dilute mRNA with serum-free and antibiotic-free medium to a concentration of 2 µg mRNA at a volume of 100 µl for each well (6well-plate)
- Pipet the 8 µl FuGENE® HD Transfection Reagent (Ratio: 8:2 Transfection reagent to mRNA) directly into the medium pipette up and down
- Incubate the transfection reagent: mRNA complex for 15 minutes at room temperature
- Add the transfection complex to the cells in a drop-wise manner
- Swirl the wells to ensure distribution over the entire plate surface
- after 4-6 hours change to medium (contains 10 % FCS, 1 % Pen/Strep) with or without cocktail
- one transfected 6 well plate incubate at 21 % O2-condition (normoxy) and a second plate incubate at 1 % 02- condition (hypoxy) at 37°C incubator

### 3) Alternative transfection with Lipofectamine™ LTX Reagent or PLUS™ Reagent (Invitrogen)

### a) Transfection FuGENE® HD Transfection Reagent (Roche)

- plate out 7000 cells in 500 µl medium per well of a 24 well-plate
- incubate over night
- for transfection: change to antibiotic free medium
- lute 0,5 µg mRNA in 25 µl serum-free and antibiotic-free medium for each well (24well-plate)
- pipette the FuGENE® HD Transfection Reagent 2 µl (Ratio: 8:2 Transfection reagent to mRNA) directly into the medium
- pipette up and down
- incubate the transfection reagent: mRNA complex for 15 minutes at room temperature
- add the transfection complex to the cells in a drop-wise manner
- swirl the wells to ensure distribution over the entire plate surface
- after 4-6 hours change to medium (contains 10 % FCS, 1 % Pen/Strep) with or without cocktail

### b) Lipofectamine™ LTX Reagent and PLUS™ Reagent (Invitrogen)

- plate out 7000 cells in 500 µl Medium per well of a 24 well-plate
- incubation over night
- before transfection: change to antibiotic free medium
- dilute 0,5 µg mRNA in 100 µl serum-free and antibiotic-free medium for each well (24well-plate)
- mix gently
- mix PLUS Reagent gently before use
- add 0,5 µl PLUS™ Reagent in the mRNA-Medium mix, mix gently and incubate 5 min at room temperature mix Lipofectamine™ LTX Reagent gently before use
- add 1,25 µl directly to the diluted mRNA); mix gently
- incubate for 30 min at room temperature
- add the 100 µl mRNA- Lipofectamine™ LTX Reagent complex
- to the well
- mix gently by rocking the plate back and forth
- incubate the cells at 37°C in the CO2 incubator for 4-6 hours
- change to medium with or without cocktail
- during the optimization of the transfection efficiency → in crease of the amount of mRNA concentration at 1 µg per well (24 well plate)
- every 72 h repeat the transfection with FuGENE® HD or Lipofectamine™ LTX Reagent and PLUS™ Reagent

### 4. Protease inhibitor treatment (MG132) to increase the half-life of the proteins produced by the transfected mRNA:

- 48h after transfection: treatment of two wells in every plate with MG-132 (10 µM/well) with and without cocktail for 6 h, than change medium

### 5. Results

Analysis of mRNA amount (per PCR) of the transfected or internal genes (oct4, nanog, hTERT, sox2, klf4) and promoter analysation (of nanog, sox, oct4)

The reprogramming results are set forht in figures 1 to 8

### 6. Tools and Methods

### 6.1 RNA-Isolation with TriFaSt™(Peglab)

- 1 ml Trifast per 10 cm² of the culture dish area
- samples can be stored for a long time at -80°C or be directly used
- samples should be kept for 5 minutes at room temperature
- addition of 0.2 ml of chloroform
- shake samples by hand vigorously for 15 seconds
- incubation for 3 minutes at room temperature
- centrifugation at 12.000 x g
- transfer the aqueous phase with RNA to new tube (save the interphase and organic phase at 4°C for the isolation of DNA and proteins).
- precipitation of the RNA with 0.5 ml of Isopropanol per 1 ml of Tri-Fast™ used for the initial homogenization.
- incubation on ice for 15 min
- centrifuge for 10 minutes at 4°C at 12.000 g
- remove the supernatant
- wash the RNA pellet twice with 75% ethanol by vortexing
- subsequent centrifugation for 8 minutes at 7,500 x g (4°C)
- remove the excess isopropanol from the RNA pellet by airdrying
- re-suspend the RNA pellet in Rnase-free water (DEPC-H20) → 30 µl - 50 µl
- dissolve the RNA pellet by passing the solution through a pipette tip several times
- incubating the solution for 10 min at 55°C
- pipetting it up and down.
- Heating the sample at 55-60°C might help to dissolve the pellet
- measure the RNA-Concentration → NanoDrop
- samples store at -80°C for 2-3 months

### 6.2 DNA-Isolation:

- Remove any left aqueous phase after RNA removal
- precipitate the DNA with 0.3 ml of 100% ethanol per ml Tri-Fast™
- mix well by inversion
- store the samples at room temperature for 2-3- minutes
- centrifugation at 2,000 x g at 4°C for 5 minutes
- move the DNA supernatant (store it at 4°C for the protein isolation)
- DNA pellet washed twice with 1 ml 0.1 M sodium citrate in 10% ethanol
- At each wash step keep the DNA in 0.1 M sodium citrate /10% ethanol for 30 minutes at room temperature (with periodic mixing)
- centrifuge at 4°C for 5 minutes at 2,000 x g
- after these two washes suspend the DNA pellet in 2 ml of 75% ethanol
- keep it at room temperature with periodic mixing for 15 minutes
- centrifuge at 2,000 x g for 5 minutes at 4°C
- dry the DNA pellet briefly for 5-10 minutes under vacuum and
- dissolve it in 8 mM NaOH by slowly passing the pellet through a wide bore pipet.
- Try to adjust the final concentration of DNA to 0.2-0.3 µg/µl with 8 mM NaOH

### 6.3 RNA treatment with DNase I (Fermentas):

- 1 µg RNA
- 1 µl 10X reaction buffer with MgCI2
- 1 µl (1 u) DNase
- to 9 µl DEPC-H2O

- incubate at 37°C for 30 minutes
- inactivation of DNase I: Add 1 µl 25mM EDTA and incubate at 65°C for 10 min

### 6.4 RT-PCR with SuperScript™ III Reverse Transcriptase (Invitrogen)

### Reaction condition:

- 1 µI oligo(dT)20 Primer
- 1 µg RNA
- 1 µl 10 mM dNTP Mix (10 mM each dATP, dGTP. dCTP and dTTP)
- to 13 µl DEPC-H20

- heat mixture to 65°C for 5 minutes
- incubate on ice for 1 min

### Add:

- 4 µl 5X First-Strand Buffer
- 1 µl 0.1M DTT
- 1 µl SuperScript™ III RT (200 units/µl)

- mix by pipetting gently up and down
- incubate at 50°C for 60 min
- inactivate the reaction by heating at 70°C for 15 minutes
- store the cDNA at -20°C

### 6.5 PCR of human oct4 (control the human fibroblasts):

Primersequence: hoct4_S: gaggatcaccctgggatatacahoct4_as: agatggtcgtttggctgaatac

Product size: 100 bp

### 6.6 PCR with Patinum Taq-Poymerase (Invitrogen)

### Preparation of PCR-reaction on ice:

- 5 µl 10X PCR Buffer
- 1 µl 10 mM dNTP mixture 0.2 mM each
- 1.5 µl 50 mM MgCI2
- 1 µl Primer mix (10 µM each) 0.2 µM each
- 1 µl Template DNA
- 0,2 µl Platinum® Taq DNA Polymerase 1.0 unit
- to 50 µl DEPC-H20

### PCR-Program (Biometra Tprofessional):

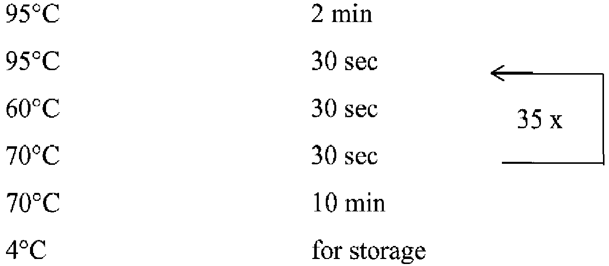

### PCR-control with 3% TAE-agarose-gel for electrophoresis

### 6.7 Protocol to produce iPScells

### 6.7.1 BD Matrigel™ hESC-qualified Matrix

- put the sterilized tip box and eppendorf tubes over night in - 20°C freezer
- thaw the Matrigel bottle on ice in the 4°C fridge overnight
- aliquot Matrigel for hESC upon arrival, store at -80°C, only thaw once and do not re-freeze to avoid breakdown of growth factors
- for coating purposes, dilute Matrigel appropriately by using serum-free medium (0.3 µg/µl for iPS, 1 µg/µl for hESC)
- add 50 µl Matrigel-dilution per cm2 growth surface
- incubate 60 min at RT under sterile conditions or overnight at 4°C
- soak off remaining Matrigel/medium
- Medium: mTeSRI (Stem Cell Technology)

Notes: Coating recommendations (0.3 µg/µl) is of course dependant on the cell type that you are using to generate iPS. It is recommended to coat (not gel!) plastics for hESC culture using 1 ug Matrigel/µl. Use plates immediately or store for max 7 days at 4°C covered with serum-free medium under aseptical conditions (sealed).

Please note that you always should process, store and use your coated Matrigel plates exactly the same way to avoid variabilities (mainly through breakdown of growth factors). In the beginning or for establishment it nessesary to titrate a coating volume and - concentration (maybe 3 dilutions 0.3, 0.6, 1 ug/ul) that suit your specific type of cells best.

### 6.7.2 BD Matrigel™ Basement Membrane Matrix, Growth Factor Reduced (GFR)

- aliquote the matrigel such as hESC-qualified Matrigel
- thaw tube overnight on ice at 4°C
- dilute with 6 ml cold basal media and mix well
- add 1 ml per well of 6 well plate
- incubate the plate at room temperature for one hour or overnight at 4°C
- plate may either be used immediately or stored at 4°C (plate are be good for least one week)
- remove the excess liquid and wash once with basal medium
- basal medium component: D-MEM/F-12; 20% KO Serum Replacer; 1% Non-essential Amino Acids; 1mM L-Glutamine, 0.1 mM 2-Mercaptoethanol; 100 ng/ml bFGF
- use mouse cells then add LIF-supernatent to the basal medium

### 6.7.3 Production of LIF-supernatant

- coat petri dish (145 mm) with 0,1 % gelantine (Bovine Type x?, Sigma)
- plate 3x10e10 LIF producing feeder cells SNL in every dish
- Medium component: D-MEM high glucose, 10% FCS, 1 % Pen/Strep; 1 % L-Glutamine
- after 24 hours collect the medium from the dish and filter through a 0,22 µm filter
- aliquote the medium containing LIF in 500 µl aliquots and store at -20°C
- change media after 24 hours or 48 hours

## Claims

1. A process for preparing reprogrammed mammalian cells *in vitro* without the use of a virus, comprising
a) providing isolated mammalian cells to be reprogrammed,
b) transfecting one or more reprogramming mRNA molecules into the cells provided in step a), wherein the reprogramming mRNA molecules are encoding at least one reprogramming protein selected from the group consisting of Ronin, Oct4, Klf4, Sox2, Nanog and TERT,
**c)** culturing the cells obtained in step b) in a cell culture medium and under a condition suitable to allow translation of the transfected reprogramming mRNA molecules, and wherein a condition suitable to allow translation of the transfected reprogramming mRNA molecules is a temperature from 30°C to 38°C and is an oxygen content in the cell culture medium from 0.5% to 21% and wherein the cell culture medium has a glucose content of 0.1 g/l to 4.6 g/l, so as to obtain reprogrammed cells, wherein said reprogrammed cells are mammalian cells exhibiting a pluripotent or multipotent stem cell character.

2. The process according to any one of the preceding claims, wherein the cell culture medium contains at least one inducing substance selected from the group consisting of reversine, resveratrol, selenium, selenium containing compounds, EGCG (epigallocatechin-3-gallate), valproic acid, salts of valproic acid and sodium valproate.

3. The process according to any one of the preceding claims, wherein the cell culture medium contains at least one transient proteolysis inhibitor.

4. The process according to claim 3, wherein the transient proteolysis inhibitor is selected from the group consisting of protease inhibitor, proteasome inhibitor and lysosome inhibitor.

5. The process according to any one of the preceding claims, wherein the cells to be reprogrammed are human cells.

6. A process for inducing the dedifferentiation of mammalian cells, wherein a process according to any one of claims 1 to 6 is carried out.

7. A process for improving the expansion of mammalian stem cells *in vitro,* wherein a process according to any one of claims 1 to 6 is carried out, comprising
a) providing isolated mammalian cells to be reprogrammed,
b) transfecting one or more reprogramming mRNA molecules into the cells provided in step a), wherein the reprogramming mRNA molecules are encoding at least one reprogramming protein selected from the group consisting of Ronin, Oct4, Klf4, Sox2, Nanog and TERT,
**c)** culturing the cells obtained in step b) in a cell culture medium and under a condition suitable to allow translation of the transfected reprogramming mRNA molecules, and wherein a condition suitable to allow translation of the transfected reprogramming mRNA molecules is a temperature from 30°C to 38°C and is an oxygen content in the cell culture medium from 0.5% to 21% and wherein the cell culture medium has a glucose content of 0.1 g/l to 4.6 g/l, so as to obtain reprogrammed cells, wherein said reprogrammed cells are mammalian cells exhibiting a pluripotent or multipotent stem cell character,
wherein said culturing in step c) is **characterized by** prolonging expansion of the pluripotent or multipotent stem cells obtained, whereby said stem cells to be expanded are kept in a dedifferentiated status.

8. A process for the rejuvenation of aged mammalian cells, wherein a process according to any one of claims 1 to 6 is carried out.
